# EUROPEAN PATENT APPLICATION

(11) **EP 1 884 274 A1**
(43) Date of publication of application: **06.02.2008**
(21) Application number: 07115559.2
(22) Date of filing: 28.12.2001
(51) Int. Cl.: B01D 15/00, A61M 1/36, B01D 15/02, B01J 41/04, C07K 1/22

(54) **Extracorporeal capturing of specific bio-macromolecular entities from extracellular body fluids**

(30) Priority: 29.12.2000 DK 200001955
(62) Divisional of application: 01272621.2
(71) Applicant: UPFRONT CHROMATOGRAPHY A/S, DK-2100 Copenhagen Ö (DK)
(72) Inventor: LIHME, Allan Otto Fog, 3460 Birkerød (DK)
(74) Representative: Plougmann & Vingtoft A/S

(57) **Abstract**

The invention describes a method and devices for extracorporeal capturing of specific bio-macromolecular entities from extracellular body fluids. The method is a column-type process and comprise the extracorporeal circulation of the extracellular body fluids through a stabilised fluidised bed of an adsorbent medium characterized by having specific affinity towards one or more specific bio-macromolecular entities.

Also described is a method comprising such extracorporeal circulation through an adsorbent medium in a continuous batch process, said adsorption medium characterised by having specific affinity towards one or more specific bio-macromolecular entities and further being characterised by comprising adsorbent particles having small diameters and high densities.

## Description

### Field of the Invention

The present invention describes a method for extracorporeal capturing of specific bio-macromolecular entities from extracellular body fluids, said method comprising extracorporeal circulation of the extracellular body fluids through a stabilised fluidised bed of an adsorbent medium characterised by having specific affinity towards one or more specific bio-macromolecular entities. The invention also relates to a method wherein the extracellular body fluids are circulated through a fluidised bed with a specific adsorbent medium consisting of small, high-density particles.
Also disclosed are methods and devices for therapeutic treatment of mammals using extracorporeal capturing of specific bio-molecular entities from extracellular body fluids.

### Background of the Invention

The concept of processing extracellular body fluids through a column of detoxicant particles for the purpose of removing specific substances from the fluid is well-known in particular in the case of human blood (Yatzidis, H.; A convenient hemoperfusion micro-apparatus over charcoal. Proc. Europ. Dial. Transplant Ass., 1:83, 1964). While the technique is initially very effective, such previous attempts at hemoperfusion have been plagued by very high losses of white cells and platelets (Dunea, G. and Kolff, W. J.; Clinical experience with the Yatzidis charcoal artificial kidney. Trans. Amer. Soc. Artlf. Int. Organs, 11:179, 1965), as well as clotting, sludging, and channelling of blood in the column. The column then becomes ineffective and the patient suffers thrombocytopenia and leucopenia. Further, fine detoxicant particles tend to be released Into the blood stream to become emboli in blood vessels and organs such as the lungs, spleen, and kidneys (Hagstam, K. E., Larsson, L. E., and Thysell, H.; Experimental studies on charcoal hemoperfusion in phenobarbital intoxication and uremia including histological findings. Acta. Med. Scand., 180:593, 1966).

Conventional therapeutic procedures for extracorporeal purification of blood Include membrane techniques such as hemodialysis, plasma pheresis, and ultrafiltration and sorption techniques such as hemoperfusion, and plasma perfusion or combinations of these methods. The mentioned membrane techniques all separate compounds comprised In the blood according to their size but does not selectively remove specified components. In contrast, sorption techniques can be both selective and non-selective.

Hemoperfusion involves the passage of the contaminated blood over a solid surface of a detoxicant particulate mass that separates the contaminant by sorption or by ion exchange. Another technique, plasma perfusion, involves separation of blood cells prior to contacting plasma with the sorbent. In any case, treated blood or combined cells and treated plasma are returned to the patient's blood circulation system.

When the specific component which is to be removed from the blood is well-defined, sorbents comprising affinity specific molecules specially designed to attract and bind the specific component may be employed. The following is examples of different sorption techniques: (1) removal of autoimmune antibodies, immunoglobulins and immune complexes using sorbents such as Protein-A; (2) removal of circulating toxins and tumour antigens (e.g., α-fetoprotein associated with hepatic cancer, carcinoembryonic antigen associated with various carcinomas, thioesterase or cytokeratins associated with breast cancer, and the like) using sorbents such as immobilised monoclonal antibodies and specific immobilised affinity specific molecules; (3) removal of protein bound toxins and drugs (e.g., in the case of psychotomimetic or narcotic drug overdose) based on the antigenic properties of these protein conjugates; (4) removal of cholesterol (low density lipoproteins, DLD) using sorbents specific to LDL; (5) removal of excess phosphate on the MgO/TiO complex deposited on active carbons; (6) adsorption of triglycerides, cholesterol and fatty acids on hydrophobic polymer materials; (7) removal of human immunodeficiency virus using calcinated hydroxyapatite-silica-alumina adsorbing materials; (8) absorbing free hemoglobin from plasma on polyphenylalanine, polyalkylene-oxide or mineral or polymeric porous materials bearing groups of tyramine, tyrosine, phenylalanine and aminophenol on the surface.

In any of the conventional hemoperfusion systems, the blood comes into direct contact with the sorbent, such as active carbon typically contained in a packed bed column, which leads to two kinds of serious problems. First, particular carbon is released into the blood stream where it causes the formation of emboll in the blood vessels and organs such as lungs, spleen and kidneys. Second, the biological defense system of blood may be activated: the blood may coagulate to form a clot, or thrombus, the immune system may be activated, and blood cells may act to encapsulate the artificial device. Indeed, while hemoperfusion on activated carbon was initially very effective in terms of removing the specific components, attempts to improve the technology have been plagued with very high loss of white cells and platelets as well as clotting, sludging and channel formation of blood in the column comprising the adsorbent. Thus, the column becomes ineffective and the patient suffers from thrombocytopenia, and severe embolia. Accordingly, it is an object of this invention to provide Improved hemoperfusion methods in such a way that very large bio-molecular entities such as specific cell types or virus can be removed from the blood.

Fluidised bed processes were originally Introduced for the recovery of antibiotics and other low molecular weight compounds from cell-containing fermentation broths. More recently, stabilised fluidised bed absorption technologies have been used for the capturing of proteins from particle-containing feedstock without prior removal of particles, thus enabling clarification of a cell suspension or cell homogenate and the concentration of the desired product in one single operation.

US 4,846,786 describes the removal of chemical species from a biological fluid in an extracorporeal device comprising an oscillating chamber containing suspended adsorbent beads.

US 4,863,611 describes an apparatus for removing material from a biological solution consisting of a reactor chamber comprising agitation means for preventing packing of adsorbent beads in the reactor and dispersing the mixture of beads and biological solution throughout the reactor chamber.

WO 00/12103 describes a method of removing HIV and other viruses from blood by circulating blood through hollow fibers which have in the porous exterior surface, immobilized affinity molecules having specificity for viral components.

US 5,641,622 describes a method combining immunoaffinity separation with continuos flow centrifugal separation for the separation of nucleated cells from a heterogeneous cell mixture. The method comprises contacting particles carrying a substance binding the desired type of cell and takes advantage of the difference in sedimentation velocity between nonbound cells and particle bound cells, exploited in a continous flow separator to achieve the desired separation. While the possibility of using particle-bound antibodies or other biologic binding substances to bind cells is demonstrated, the separation principle comprises continuous flow centrifugation which is technically demanding and dependant on expensive equipment and thus not readily amenable for extracorporeal adsorption methods.

US 6,051,146 describes a fluidised bed of particles able to retain second particles within a rotating fluid chamber. The separation is achieved by centrifugation.

US 6,153,113 describes a system for separation of particles having different sedimentation velocities namely particles bound together by ligands attached covalently to the first particle and binding the second particle by noncovalent forces. Separation is achieved by centrifugation.

WO 00/56982 describes an expanded bed procedure utilized as a first capture step in the processing of bio-macromolecules including nucleic acids, viruses and bacteria. Extracorporeal treatment of body fluids is not disclosed nor are any therapeutic methods or therapeutic devices anticipated or disclosed.

US 5,935,442 discloses in details a fluidised bed chromatographic process for the purification of various molecules. While this invention describes the numerous methods for tailoring adsorbent beads for chromatographic uses US 5,935,442 does not disclose methods for therapeutic extracorporeal treatment of whole blood and other body fluids.

US 5,522,993 discloses chromatographic beads for downstream processing, especially stabilised fluidised bed separations. Beads are described as spherical, having densities in the range 1.1 to 1.5 g/ml and diameters between 100 and 1000 µm (125-315 µm). Very small and very dense particles are not disclosed, and the use of stabilised fluldised bed separation for extracorporeal therapy or devices for therapeutic application are not mentioned.

US 4,846,786 and US 4,863,611 both describe an apparatus for efficient agitation of beads and used for treatment of blood in a extracorporeal contacting procedure. Fluidization of adsorbent beads is achieved by a specific geometry and agitation of the reactor chamber. These pieces of prior art describes particles with diameters 10-400 µm and being kept in place by an appropriate mesh, preferably a 40 µm pore mesh keeping back 200 µm particles. Thus this Invention is not suitable for adsorption of larger bio-macromolecular entities such eucaryotic cells, e.g. blood cells, foetal cells.

US 5,759,793 describes a method for selection of specific mammalian cell types in a mammalian cell population by means of a magnetically stabilised fluidised bed. While separation and purification of hematopoietic cells from blood are described, the invention does not describe a method or devices for continuous extracorporeal therapy.

### Summary of the Invention

Thus, the problem behind the present invention is to provide means for extracorporeal treatment of blood and other body fluids that is practicable in everyday clinical practice.

Another aim of the present invention is to provide extracorporeal therapeutic methods and therapeutic devices based on efficient adsorption of harmful substances from extracellular body fluids.

The present invention provides a relatively simple stablised fluldised bed adsorption method based on an adsorption medium consisting of adsorption particles having a specific affinity for the specific bio-macromolecular entities in a column-type container. The method may alternatively be conducted in a stirred or rotated container in which small, high density particles are utilised. Importantly, the method does not comprise a continuous centrifugation process.

Characteristic for stabilised fluidised bed adsorption is the expansion of the bed upon application of a liquid, typically in an upward flow through the bed. The space between the adsorbent particles, the void volume, is thereby increased allowing large bio-macromolecular entities contained in the sample to pass through without clogging the column. It has been found that this property makes fluidised bed adsorption particular suitable in connection with separation of specific components from extracellular body fluids, which comprise many different components, e.g. cells. Also, in a stabilised fluldised bed the liquid is passed through the column as a plug flow substantially without turbulence and back-mixing.

The fact that the extracellular body fluids in a stabilised fluidised bed are passed through a bed consisting of adsorption particles substantially without turbulence and back-mixing provides an efficient and gentle contact with a large surface area of an adsorption medium ensuring that the majority of the bio-macromolecular entities which are to be separated from the body fluid are detained. It is well known from packed bed column plug flow that elimination of turbulence, back-mixing and channel formation provide a very efficient adsorption method due to the high number of theoretical plates formed.

Optimal performance of the disclosed stabilised fluidised bed capture of specific bio-macromolecular entities from extracellular body fluids is further ensured by providing a very large surface area of the adsorbent particles (to accomplish efficient and high capacity adsorption) combined with a large density difference between the density of the body fluid and the density of the adsorbent particles (to accomplish an acceptable flow rate through the column).

The optimal density difference between the body fluid and the adsorbent particles is obtained by providing adsorbent particles having a very high density (e.g. significantly higher than the density of water). Thus high density adsorbent particles will sink in the body fluid. However, it should also be mentioned that a stabilised fluidised bed can be created by applying a downward flow of liquid to a bed of particles having densities and/or sizes allowing them to float in aqueous buffers.

Thus, the present invention provides a method for extracorporeal capturing of specific bio-macromolecular entities from extracellular body fluids, said method comprising the steps of:
(a) obtaining the extracellular body fluid from a mammal;
(b) contacting the obtained extracellular body fluid with a stabilised fluidised bed of adsorption particles characterized by having a specific affinity for the specific blo-macromolecular entities; and

(a) reinfusion of the treated extracellular body fluid into the same mammal;
wherein steps (a) and (c) are performed in a continuous process.

The present invention also provides a method for extracorporeal capturing of specific bio-macromolecular entities from extracellular body fluids, said method comprising the steps of:
(a) obtaining the extracellular body fluid from a mammal;
(b) contacting the obtained extracellular body fluid with an particulate adsorption medium characterized by having a specific affinity for the specific bio-macromolecular entities, said adsorption medium being in the form of particles having a density of at least 1.3 g/ml and a mean diameter in the range of 5-1000 µm, such as a density of at least 1.5 g/ml and a mean diameter in the range of 5-300 µm, preferably a density of at least 1.8 g/ml and a mean diameter in the range of 5-150 µm, and most preferred a density of more than 2.5 g/ml and a mean diameter in the range of 5-75 µm,
(c) reinfusion of the treated extracellular body fluid into the same mammal;
wherein steps (a) and (c) are performed in a continuous process which does not comprise a continuous centrifugation process.

### Definitions

In the present context, the term "bio-macromolecular entity" is an entity of biological origin with a size or molecular weight of at least 1,000 D, such as at least 5,000 D, preferably at least 100,000 D and most preferably at least 5,000,000 D. By definition the term "bio-macromolecular entity" also comprises any of the following entities: microorganisms and cells such as: cancer cells, certain blood cells, foetal cells, parasites, bacteria (both eu- and archaeo-bacteria) and virus. Furthermore nucleic acids e.g. transposons, retrotransposons and plasmids; polypeptides and proteins such as: prions, antigens, antibodies, hormones, certain signaling macromolecules and also organic toxins and inorganic toxins are described by the term "bio-macromolecular entity".

In the present context, the term "cell" describes the smallest unit retaining the fundamental properties of life, and comprising both prokaryotic, eukaryotic and mesokaryotic cells as well as cells from multicellular and single cellular organisms. In particular free circulating cancer cells, cancer cell precursors, certain blood cells and virus or otherwise infected cells are considered Interesting in relation the present invention.

In the present context, the term "microorganism" describes very small organisms belonging to various groups, most relevant to the present invention are microorganisms belonging to the groups of: protozoa, fungi and bacteria. However also certain plants e.g. algae are considered as micro-organisms. Although characterised by a non-cellular structure virus, and sometimes also prions are considered as micro-organisms.

In the present context, the term "virus" describes an infectious agent of small size and simple composition that can multiply only in living cells of animals, plants, or bacteria. They consist of a nucleic acid (DNA or RNA for the retroviruses) and of a proteinic coat.

The term "viroids" refer to infectious particles smaller than any of the known viruses. These particles consists only of an extremely small circular RNA (ribonucleic acid) molecule and lack the protein coat of a virus. Although viroids at present only are known as agents of certain plant diseases it is possible that certain diseases in mammals could be caused by viroids. This is also the case with two other types of "infectious" nucleic acid molecules the transposons and the retrotransposons.

The term "transposon" refer to a DNA sequence that is able to insert itself at a new location in the genome. Transposons are typicallly linear in structure, contains only one or a few genes and have special sequence structures (inverted repeats) at Its ends.

The term "retrotransposon" refer to a transposon that mobilizes via an RNA form; the DNA element is transcribed into RNA, and then reverse-transcribed into DNA, which is inserted at a new place in the genome.

The term "plasmid" refer to an autonomous self-replicating extrachromosomal circular DNA.

In the present context, the term "prion" describes an aberrant form of a normally harmless protein found in mammals and birds. The normal form of the protein is located on the surface of cells in the brain. The molecular weight of a protease-treated abnormally folded and pathogenic prion protein is 27-35 kD. Only when it is in the aberrant configuration does the prion protein cause disease. The pathogenic protein can enter the brain through infection, or it can arise from a mutation in the gene that encodes the protein. Once present in the brain it multiplies by inducing benign proteins to refold into the aberrant shape. The normal protein conformation can be degraded rather easily by proteases, but the aberrant protein shape is more resistant to this enzymatic activity. Thus, as the prion proteins multiply they are not broken down by proteases and instead accumulate within nerve cells, destroying them. Progressive nerve cell destruction eventually causes brain tissue to become riddled with holes in a spongelike, or spongiform, pattern. Diseases caused by prions include five disorders that affect humans: Creutzfeldt-Jakob disease, new variant Creutzfeldt-Jakob disease, Gerstmann-Sträussler-Scheinker disease, fatal familial insomnia, and kuru. Other prion diseases, such as scrapie, bovine spongiform encephalopathy (commonly called mad cow disease), and chronic wasting disease of mule deer and elk, are suffered by animals. (ref. Encyclopædia Britannica, Weber EL. Rev Argent Microbiol 1999 Oct-Dec;31(4):205-18 and Horiuchi M EMBO J 1999 Jun 15;18(12):3193-203).

The terms "polypeptide" and "protein" are almost synonymous both describing molecules mostly consisting of amino acids polymerised by peptide bond formation. Normally the expression "polypeptide" is used on relative short chains of polymerized amino acids whereas the term "protein" is used to describe larger molecules.

The term "antigen" is a substance which is reactive with a specific antibody or T-cell.

The term "antibody" refer to a protein (immunoglobulin) produced by B lymphocyte cells that recognises a particular "antigen"

In the present context, the term "affinity specific molecules" describes molecules which are characterised by their ability to associate specifically with a specific blo-macromolecular entity or part of that entity under physiological conditions. Examples of "affinity specific molecules" are: specific binding gelatins, albumins, hemoglobulins, immunoglobulins Including poly- and mono clonal antibodies, antigens, protein G, protein A, blood group specific oligosaaccharides, lectins, glycoproteins such as ovomucoids or specific binding parts thereof, biotin binding proteins, avidin and streptavidin, enzymes, proteases, receptors and protease inhibitors; specific binding parts of the above mentioned classes of molecules as well as mixtures of these.

In the present context, the term "extracellular body fluids" describes the extracellular fluids of the mammalian organism. Examples are: blood, ascites, plasma, lymph, amnion fluid, urine, saliva, and cerebrospinal fluid.

In the present context, the terms "adsorbents", "particulate material" and "adsorption medium" are used synonymously.

In the present context, the term "adsorption medium" refer to an adsorption medium consisting of particles that are able to adsorp specific bio-macromolecular entities. The terms "adsorption medium" and "adsorption particles" are used synonymously.

A "fluidised bed" is herein defined as any arrangement of agitation, buffers and adsorbent particles in which a space between the individual particles wider than the minimum space obtained in a packed column is achieved.

Thus, according to this definition, any set of adsorbent particles that are utilised in any type of non-packed bed reactor constitutes a "fluidised bed". Examples of such fluidised beds are fluidised beds obtained by applying fluid flow to an initially packed bed of adsorbent particles at flow rates high enough to effect an expansion and "fluidisation" of the bed as described in chemical engineering textbooks (e.g. H. Scott Fogler in "Elements of Chemical Reaction Engineering", p. 786, Prentice-Hall PTR, 1999) as well as continuous-stirred tank reactors (CSTR's) wherein "fluidisation" is achieved by a combination of a continuous fluid flow and an efficient mixing of the adsorbent bed system (see e.g. H. Scott Fogler in "Elements of Chemical Reaction Engineering", p. 10, Prentice-Hall PTR, 1999).

A "stabilised fluidised bed" is defined as a fluidised bed in which there is a low degree of back-mixing of the adsorbent particles as a consequence of the movement of each particle being restricted to move within a limited volume of the total bed volume. This means that each particle is has a low extent of axial dispersion and does not have the same probability of being found at any position within the confined space of the fluidised bed. A stabilised bed thus may be characterised as having a non-homogenous composition of the entire fluidised bed as the absence of back-mixing precludes mixing of mutually heterogenous zones of the bed.

By the term "expanded bed" is meant a stabilised fluidised bed of adsorbent particles created by applying an upward liquid flow of sample or of aqueous buffer through an inlet at the bottom of a column containing the bed of particles, said particles having a density and/or size distribution that make them position within a confined space of the fluidised bed. Like a stabilised fluidised bed, an expanded bed is characterised by having a low degree of back-mixing of the adsorbent particles. With the exception of magnetically stabilised fluidised beds, the terms "expanded bed" and "stabilised fluidised bed" are to a large extent synonymous.

By the term "magnetically stabilised fluidised bed" is meant a stabilised fluidised bed of adsorbent magnetizable particles obtained by placing adsorbent particles in a radially uniform magnetic field parallel to the path of fluid flow through the bed.

In the present context, the term "expanded bed adsorption" describes the particular chromatographic technology wherein an adsorbent medium contained in a column having an inlet and an outlet is allowed to rise from its settled state by application of a fluid stream of e.g. the sample (body fluid) or an aqueous buffer in an upward flow, thereby increasing the space between the adsorbent particles. This can happen simultaneously with or prior to the introduction of the fluid sample.

By "continuous process" is meant a process that can be defined by a constant function being applied at the starting time point of the process and terminated at the end point of the process. Thus, in the present context a typical example of a continuous process is a procedure in which a certain type of body fluid, typically blood, is removed at a constant flow (i.e. substantially uninterrupted flow) from a patient and also reintroduced into the patient with a similar constant flow.

In other words it is to be understood that the removal of blood from the patient at a given flow rate, the contacting of the blood with the adsorbent and the reintroduction of the blood into the patient is performed in one consecutive and interrelated procedure at the patients bed site.

This procedure is to be understood in contrast to any other 'discontinuous' procedure wherein the body fluid is withdrawn from the patient in one independent procedure at one time, optionally stored and contacted with an adsorbent in a batch-wise manner at another time and reintroduced into the patient at still another time chosen largely independent of the two first procedures.

A "stirred tank" is an arrangement in which adsorbent particles are kept in free suspension by agitation, rotation or any other known method to make them available for interaction with non-clarified solutions containing particulated matter which would not be possible in a packed bed without a pre-filtration or a pre-centrifugation step. Such stirred tank methods are characterised by the interaction between sample substances and the adsorbent beads being a single-state adsorption procedure which is less efficient than a multi-stage adsorption process as it occurs in a packed (or stabilised fluidised) bed. In this respect a non-stabilised fluidised bed resembles a stirred tank suspension due to the back-mixing (turbulence) and channeling observed in such fluidised beds. By contrats a stabilised fluidised bed combines the multi-stage adsorption characteristics of packed beds with the ability to cope directly with viscous and/or particulate samples without the need for pretreatment steps as filtration and centrifugation.

The number of "theoretical plates" in a chromatographic system is an expression of the number of equilibria that can be formed between the adsorbent beads and the sample component interacting with the bed of adsorbent beads. This number is expressed in number per meter column and can be calculated from the residence time of a suitable tracer being pumped through the column as known to a person skilled in the art, see e.g. The Amersham Biosciences booklet "Expanded Bed Adsorption Handbook, Ref. no. 18112426", which is available at http://www.chromatography.apbiotech.com, and furthermore is incorporated herein by reference.

In the present context the expression "conglomerate" is intended to designate a particle of a particulate material, which comprises beads of core material of different types and sizes, held together by the polymeric base matrix, e.g. a particle consisting of two or more high density particles held together by surrounding agarose (polymeric base matrix) as described in WO 92/18237 and WO 92/00799. The expression "pellicular" is intended to designate a composite of particles, wherein each particle consists of only one high density core material coated with a layer of the polymeric base matrix, e.g. a high density stainless steel bead coated with agarose.

In the present context the "density" of particles is the density of particles in the hydrated state.

### Detailed Description of the Invention

The present invention relates to a method for extracorporeal capturing of specific bio-macromolecular entities from extracellular body fluids, said method comprising the steps of:
(a) obtaining the extracellular body fluid from a mammal;
(b) contacting the obtained body fluid with a fluidised bed of an adsorption medium characterized by having a specific affinity for the specific bio-macromolecular entities;
(c) reinfusion of the treated body fluid into the mammal;
wherein steps (a) and (c) are performed in a continuous process.

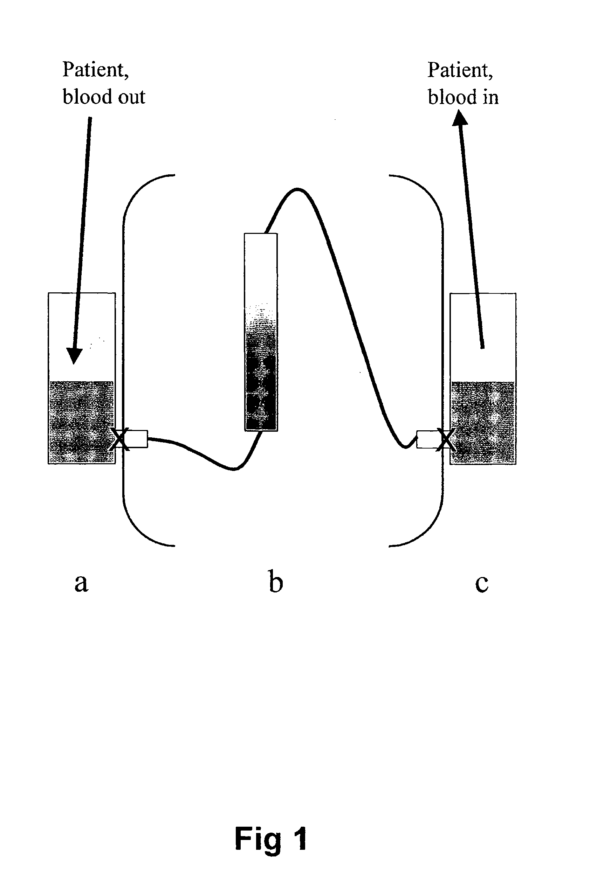

Figure 1 illustrates the general set up. By adjusting the volumen of the receiving (a) and the delivering (c) reservoirs to the flow of the body fluid it is possible to operate the whole process as a continous process while still allowing sufficient time for the body fluid to contact the adsorption medium. It is understood that the withdrawal of body fluids should take the physiological limitations of the specific mammal into account ensuring that the mammal is unharmed by the treatment.

As illustrated in example 1, the special properties of stabilised fluidised beds make it possible to pass even such complex cell suspensions as whole blood through the column. This opens the possibility for the use of fluidised bed adsorption for therapeutic extracorporeal depletion of specific bio-macromolecular entities cells from body fluids, especially from blood and for retrieving other substances from body fluids without introducing filtration steps, as illustrated in example 2. This technique offer several advantages over present day techniques for the removal of specific cell types such as differential centrifugation techniques, cell sorting techniques or techniques based on injection of so-called humanized (non-immunogenic) reagents. In comparison with such techniques the extracorporeal depletion of specific cells is simple and rugged. It allows the use of therapeutic reagents that when introduced into the body will cause undesirable immunogenic reactions since said reagent molecules are not introduced into the body. This is a major advantage in comparison with the present use of antibodies and receptor-constructs for injection. Thus, antibodies and other receptor molecules that are known from *in vitro* experiments to possess the desired binding capabilities can be used directly after immobilization. If immobilization is based on streptavidin-biotin binding, a flexible approach is ensured because the receptor molecule will be applicable simply after biotinylation.

Furthermore, the particles described herein can be used for analytical purposes. One illustrative example being the preparation of a suitable sample by catching and concentrating trace-level analytes from a large amount of blood by passing said volume of blood trough a specifically binding stabilised fluidised bed, followed by elution of the bound substance for subsequent analysis, or, in a particularly preferred embodiment, followed by the analysis for the target analyte directly in its bound state on the particles.

Safety is another Important advantage of the method of removing cells by extracorporeal depletion of the specific cells from body fluids by passage over a fluidised bed. This method eliminates the risk of spreading e.g. virus when cells are lysed by antibodies that are injected and which upon binding their cellular targets activate complement. Prior use of extracorporeal circulation methods for removal e.g. of antibodies by column adsorption, exchange of plasma (plasmapheresis), and to a certain extent hemodialysis methods all support the feasibility and relative safety of the approach.

Finally also the efficiency and capacity of the method should be considered. The passing of a whole cellular compartment of blood through a specific adsorbent matrix is much more efficient for removing specific cells since much higher relative concentrations of antibodies or other receptors can be used than when injecting the targeting reagents. Also, the reagents are not destroyed by the degradative systems and clearance mechanisms present in the body because the agents are immobilized (and thus stabilised) and are furthermore in contact with the components of the blood for only a short time.

Extracorporeal adsorption is a method of choice compared to inhibition of an undesired reaction in the body by simple injection of antibodies and/or specific inhibitors (antagonist ligands) when:
- binding of the antibody/inhibitor does not efficiently block the detrimental activity, e.g. when the binding affinity is to low to ensure inhibition
- blocking substance is toxic to the patient or gives rise to undesired reactions (e.g. immune responses) in the body
- when there is a need to remove whole cells or very large biomolecular substances.

Extracorporeal therapy is also an alternative to plasma exchange therapy. With this latter method there is a need for expensive plasma and/or plasma fractions, beneficial components are also removed from the patient together with the undesired components and there is a danger of transferring infectious substances to the patient (e.g. virus such as hepatitis virus and HIV).

Current extracorporeal capture methods and devices are slow, characterized by discontinuous uses involving filtration and/or centrifugation steps, have low capacities due to clogging of filters and very few examples have been demonstrated of the capture of whole cells.

This has precluded the wide-spread use of an otherwise very promising and supplementary therapeutic technology.

Membrane-based methods and hemoperfusion on activated carbon and ion exchange resins are well-known but are characterised by non-specific binding properties and therefore concurrently remove beneficial proteins and substances that therefore need to be replaced. Soft gels with affinity ligands give good selectivity but lead to difficulties with clogging and poor flow rates as they will collapse in the packed bed state.

Blood is especially difficult to process in an extracorporeal treatment device as blood is viscous and will coagulate if not counteracted by additives and contains high numbers of different cells that are very fragile and high concentrations of proteins (80 mg/ml). Furthermore it is extremely critical that nothing of a particulate nature is introduced into the bloodstream of the patient undergoing extracorporeal therapy.

Devices have been described that keep adsorbent particles in free suspension by intensite agitation (US patent 4,846,786 and 4,863,611) but it is not disclosed how blood cells will survive the shear forces occurring in such devices in an intact state.

Examples of extracorporeal methods and adsorbents include specific adsorption of lipoproteins on porous, hard particles (US 4,656,261) . This was only shown to work however in a stirred batch experiment. In another example of prior art, a method based on plastic (particles, film or hollow fiber) coated with albumin (US 6,090,292) was diclosed. This method takes advantage of the fact that albumin can be used as a ligand for detoxifying blood or plasma with a high number of important bacterial toxins and medication substances. There is no teaching however, on how to construct a perfusable packing from 10-500 micrometer (diameter) beads and instead examples of batchwise adsorption of whole blood are disclosed, while examples with packed columns comprise plasma pumped at a flow rate of only 2 ml/mln and heparinised whole blood perfused at 0.5 ml/min only. US 5,041,079 teaches removing agents for treatment of patients harbouring the human immunodeficiency virus and the use of plasma instead of blood is recommended. US 5,258,503 discloses components in an extracorporeal system for removing autoantibodies, said system incorporating filters to separate particulate material from the soluble components of blood and using porous and hard particles as adsorbents.

US 4,865,841 describes removing unwanted antibodies by contacting them with immobilised antigen (silica) in order to allow therapeutic immunotoxins to exert their effect. Removal was affected by passage of plasma (prepared by hollow fiber filtration) which could be pumped at 20-25 ml/min. Other devices for keeping beads in suspension (fluidised bed) include the Taylor-Couette flow device by Ameer et al. (Biotech. Bioegn. 62, 602-608, 1999) which is however also characterised by a substantial problem with shear which led to a high degree of hemolysis.

An ideal device for therapeutic extracorporeal treatment of blood will therefore be small and continuously operated. For extracorporeal adsorption processes this demands adsorbent materials with high capacities. This can be achieved with adsorbent beads as opposed to adsorbent hollow fibers and sheets but it has not been possible until now to use adsorbent beads to treat blood because of the packing of the beads in conventional column arrangements.

The present invention combines the use of high-capacity bead-formed adsorbent materials with the possibility of performing real hemoperfusion on whole stabilised blood.

The body fluids are obtained, handled and reinfused by methods and utensils known to a person skilled in the art. Evidently, the method will depend on the body fluid and the condition to be treated. It is understood that the entire method is performed under aseptical conditions. As an example with regard to blood a needle is introduced into e.g. a peripheral vein connected via a suitable tube to the container containing the fluidised absorption medium and reinfused into the patient via a inlet tube connected to a needle inserted into another vein. In situations where large volumes are to be withdrawn form the mammal, blood may be withdrawn from vena subclavia.

Optionally, an anticoagulation substance such as sodium citrate, heparin or dextran can be added to the blood when withdrawn from the body to prevent coagulation of the blood. Dextran reduces the viscosity of the blood and, in combination with addition of saline, ensures an increased distance between the blood cells and the blood plates. Such anticoagulants may be added in quantities sufficient for non-coagulation of the blood.

Before reinfusion of the treated blood into the mammal the anticoagulation effect of e.g. heparin, may be reduced with the right amount of a number of substances such as heparinase, protamine and/or vitamin K.

To avoid embolism, great precaution has to be taken to avoid adsorption medium particles to enter the patient upon reinfusion. In the preferred embodiment of the present invention, the adsorption medium comprises very dense particles which to a large part eliminate the problem. However, as a protective measure, a particle capture device preferably is employed downstream of the adsorption medium container to remove any residual particles from the remainder of the body fluid before it is returned to the patient. The particle capture device may be a filter or mesh having openings of a size that retain any particulate material of the adsorption medium while letting the non-adsorbed entities of the body fluid pass through. Unfortunately, an effective particle trap based on a filter or a mesh may have an effective pore-size of a dimension that either retain or impose detrimental effects on certain elements of whole blood.

In a particular embodiment of the invention the adsorption medium consists of conglomerate or pellicular particles comprised of a polymer matrix into which at least one density controlling particles have been incorporated. Said density controlling particles can be made by ferro- or para-magnetic material. Such ferro- or para-magnetic particles can be used in combination with a magnet to ensure that no beads are entering the patient when returning the body fluid.

A fluidised bed is created when a liquid, e.g. a body fluid or an aqueous buffer is passed upwards through a bed of adsorbent particles with sufficient velocity wherein the drag (frictional) lifting force of the liquid counterbalance the gravitational forces on the adsorbent particles thereby obtaining a steady state in which the the bed is expanded and the space between the adsorbent particles are increased . A sufficient and steady velocity of the liquid may be obtained by a suitable pump as will be understood by the person skilled in the art. In a fluidised bed the particulate material is completely submerged and levitated in the fluidizing fluid.

A stabilised fluidised bed, however, is further characterized by a low degree of back-mixing of the adsorbent particles. This means that each adsorbent particle moves within a limited volume of the total fluidised bed volume. This also means that a stabilised, fluidised bed that contain a non-homogenously distributed population of adsorbent particles may be created.

Stabilization of a fluidised bed can been obtained by different means.

In expanded bed adsorption, stabilization of the fluidised bed is obtained by use of adsorption particles having a well-defined size distribution as well as a well-defined density distribution together with a column designed to give an even liquid flow distribution. The stability arises when the adsorbent particles make up a so called classified (or stratified) bed where the larger and/or most dense adsorbent particles are positioned closer to the bottom of the bed and the smaller and/or less dense adsorbent particles are positioned closer to the top of the bed in an upward-flow system. The bed expands as the adsorption particles are lifted by the upward liquid flow through the column. As a matter of example illustrating the principle of formation of fluidized beds such beds can also be created by applying a downward flow of liquid to a bed of particles having densities and/or sized allowing them to float in aqueous buffers. Further information about expanded bed adsorption technology can be found in the book "Expanded Bed Adsorption Handbook" by Amersham Biosciences, which is available as a PDF file at http://www.chromatography.apbiotech.com by referring to ref. no.: 18112426.

In magnetically stabilised fluidised bed chromatography stabilization of the fluidised bed is obtained by placing magnetizable adsorbent particles in a radially uniform magnetic field parallel to the path of fluid flow through the bed. The effect of the magnetic field can be viewed roughly as creating a magnetic dipole in each particle of the adsorbent material, which causes it to become "sticky" in a direction parallel to the magnetic field lines. This produces what amounts to the formation of chains of beads parallel to the axis of the bed.

Expansion of the particulate materials (absorbents) inside a reactor gives the possibility to handle non-separated extracellular body fluids. In expansion the adsorbents are lifted up by an upward flow of the body fluid. The expanded volume between the fluidised adsorbents allows macromolecules comprised in the body fluid to pass through the reactor without clogging the system. Thus, adsorbent particles having a density larger than the fluid and moving downwards due to gravity may be kept in a free, fluidised state by an upward flow of liquid.

In the present invention the adsorbent medium constitutes a scavenger retaining specific bio-macromolecular entities from non-separated extracellular body fluids, e.g. whole blood. Essential blo-macromolecular entities of the extracellular body fluid, e.g. blood cells, are let through without blocking the bed due to the increased space between the fluidised adsorbent particles.

### Expanded bed adsorption

In the preferred aspect of the invention, the method is conducted in a stabilised fluidised bed comprising an expanded bed wherein the adsorbent medium is specially designed for use in an expanded bed, e.g. as illustrated in WO 00/57982, the disclosure of which is incorporated herein by reference.

Said adsorbent medium typically has a density of 1.3-20 g/ml, such as at least 2.0, at least 3.0, at least 3.5 and preferably 4.0-16 g/ml.

It is believed that the relatively small diameter of the particles combined with the high density play an important role for the capturing of bio-macromolecular entities in the stabilised fluidised bed processes. Thus, the average diameter of the particles of the particulate material is preferably 5-75 µm, such as in the range of 10-60 µm, such as in the range of 12-49 µm, more preferable in the range of 20-40 µm and even more preferable in the range of 10-30 µm.

Furthermore, it is believed that a relatively narrow particle size distribution is advantageous (bearing in mind that a certain width of the size distribution is advantageous when the material is to be use In a fluidised bed set-up), thus, it is believed that at least 95% of the particles should have a diameter In the range of 5-80 µm, such as 15-45 µm, preferably in the range of 20-40 µm.

Said adsorbent medium is typically in the form of particles having a density of at least 1.3 g/ml and a mean diameter in the range of 5-1000 µm, such as a density of at least 1.5 g/ml and a mean diameter in the range of 5-300 µm, preferably a density of at least 1.8 g/ml and a mean diameter in the range of 5-150 µm, and most preferred a density of more than 2.5 g/ml and a mean diameter in the range of 5-75 µm.

The high density is primarily obtained by inclusion of a high proportion of a dense core material, preferably having a density of at least 3.0 g/ml, such as at least 5.0, preferably in the range of 6.0-16.0 g/ml. This will result in particles which are pellicular or a conglomerate in composition. Examples of suitable core materials are inorganic compounds, metals, elementary non-metals, metal oxides, non-metal oxides, metal salts, metal alloys, and tungsten carbide, etc. as long as the density criterium above is fulfilled.

It is preferred that the core material of at least 95% of the particles is a steel bead having a diameter in the range of 2-40, such as 8-28 µm, preferably 5-25 µm.

In another embodiment the core material of at least 95% of the particles is a tungsten carbide particle having a diameter in the range of 2-40, such as 15-38 µm, preferably 5-30 µm.

Furthermore, it is preferred that at least 95% of the particles comprises one core material having a diameter which is at least 0.70 time, such as at least 0.80 time or at least 0.85 time the diameter of the particle.

Alternatively, the core material is constituted by more than one bead, e.g. beads having a diameter of less that 10 µm.

Typically, the core material constitutes 10-99%, preferably 50-95%, of the volume of the particles, and the polymer base matrix constitutes 1-90%, preferably 5-50%, of the volume of the particle.

When the core material of a large proportion of the particles (>95%) is constituted by one bead, the polymeric base matrix is typically less than 50 µm in thickness. "Thickness" is defined as the geometrical distance between the core material and the surface of the particle. The thickness is preferable less than 20 µm, even more preferable less than 10 µm, and most preferable less than 5 µm in thickness. In one embodiment, it is envisaged that the polymeric base matrix may constitute a mono molecular layer covering the core material. Thus, in this instance, it is contemplated that the polymeric matrix may be replaced with low-molecular weight species having a predominant affinity for the core material. This affinity between the low-molecular species and the core material may be improved by surface treatment of the core material, e.g. by organosilylation of ceramic materials. The monomolecular layer may also be covalently coupled to the surface of the core material by chemical means as appreciated by those skilled in the arts of chemistry.

A very important feature of the particulate material is the fact that the polymeric base matrix comprises either chargable pendant groups or affinity specific molecules for a blo-macromolecular entity as pendant groups. It is also possible to combine the two sub-types of pendant groups. It is currently preferred that the affinity specific molecules are selected from the group consisting of interchelating affinity specific molecules, hydrophobic affinity specific molecules, specific binding gelatins, albumins, hemoglobulins, immunoglobulins including poly- and mono clonal antibodies, antigens, synthetic peptides, protein G, blood group specific oligosaaccharides, lectins, glycoproteins such as ovomucoids or specific binding parts thereof, biotin binding proteins, avidin and streptavidin, enzymes, proteases, various receptors and protease inhibitors, sequence specific affinity specific molecules such as oligonucleotides, and other bio-catalysts; specific binding parts of the above mentioned classes of molecules as well as mixtures of these. These affinity ligands seem particular relevant for the binding of specific cells, virus, bacteria and other bio-macromolecules.

The chargeable pendant groups comprise moieties selected from the group consisting of polyethyleneimine, modified polyethyleneimine, poly(ethyleneimine/oxyethylene), quaternary aminoethyl (QAE), diethylaminoethyl (DEAE), sulfuric acid, phosphoric acid, and carboxylic acid. Such groups may be linked to the polymeric base matrix by means of a divinyl sulphone or a epichlorohydrin linker or by other means of linking known by those skilled in the art e.g. by using the chloride corresponding to the group. The first-mentioned possibility is particularly relevant for chargeable moieties like polyethyleneimines, modified (e.g. alkylated) polyethyleneimines and poly(ethyleneimine/oxyethylene)s. The corresponding chloride is especially relevant for chargeable groups like quaternary aminoethyl (QAE) and diethylaminoethyl (DEAE).

In one embodiment of the invention nucleic acid (such as DNA, e.g. plasmid DNA) may be captured by using chargeable pendant groups such as DEAE, polyethyleneimine (PEI) and QAE. The interaction between the negatively charged phosphate groups in the DNA molecule and positively charged amino groups In DEAE, PEI and QAE result in capturing of the nucleic acids on the particular materials while the body fluid passes through.

For the binding of bio-macromolecular entities to the surface of the particulate material, a large number of different affinity specific molecules as known by the person skilled in the art and may be employed by coupling to the polymer phase, directly or by the below-mentioned activating groups. Positively charged ion exchange affinity specific molecules are well suited for the binding of nucleic acids such as DNA and RNA, well known affinity specific molecules being DEAE, QAE, PEI, and other amino group containing affinity specific molecules. However, also interchelating affinity specific molecules may be employed as well as sequence specific affinity specific molecules such as complementary DNA/RNA strands and artificial oligonucleotides such as peptide nucleic acid (PNA) and locked nucleic acid (LNA).

Polyclonal and monoclonal antibodies, synthetic peptides and other synthetic chemical oligomers, lectins, carbohydrates, hydrophobic affinity specific molecules, enzymes and other bio-catalysts may also be relevant for the binding of virus, bacteria and other bio-macromolecular entities.

Such affinity specific molecules, like the chargeable moieties, may be linked to the base matrix by methods known to the person skilled in the art, e.g. as described in "Immobilized Affinity Ligand Techniques" by Hermanson et al., Academic Press, Inc., San Diego, 1992, which is incorporated herein by reference. In cases where the polymeric base matrix do not have the properties to function as an active substance, the polymeric base matrix (or matrices where a mixture of polymers are used) may be derivatised (activated) to form a reactive substance that can react with functional chemical groups forming a chemical covalent bond under appropriate circumstances. Thus, materials comprising hydroxyl, amino, amide, carboxyl or thiol groups may be activated or derivatised using various activating chemicals, e.g. chemicals such as cyanogen bromide, divinyl sulfone, epichlorohydrin, bisepoxyranes, dibromopropanol, glutaric dialdehyde, carbodiimides, anhydrides, hydrazines, periodates, benzoquinones, triazines, tosylates, tresylates, and/or diazonium ions.

In a preferred embodiment, the pendant group is a polyethyleneimine chain, more preferably a polyethyleneimine chains having an average molecular weight of at least 10,000 D, such as 50,000-2,000,000 D.

It is also preferred, irrespective of whether the pendant group is a polyethyleneimine, that the pendant groups form a tentacular structure on the surface of the particle. A tentacular surface structure is preferred to increase the surface area and/or the binding sites for large blo-macromolecular entities themselves. It should be understood that moieties like polyethyleneimine may form a coiled structure which is believed to facilitate the adsorption of bio-macromolecular entities. Other affinity specific molecules suitable for capture of bio-macromolecular entities such as plasmid DNA are the DEAE and QAE. They themselves do not form a tentacular structure when coupled to an adsorbent medium. In combination with spacers, e.g. dextran and other polymers, affinity specific molecules like DEAE and QAE can, however, form a tentacular structure with enhanced surface area. Also other low molecular weight affinity specific molecule, e.g. oligonucleotides, can be useful, in particular in combination with a tentacular surface structure.

In the case of immobilized enzymes or other bio-catalysts it may also be advantageous to utilize a covalent chemical coupling method known by those skilled in the art to attach the enzyme or other blo-catalyst to the material.

One important group of methods comprises derivatising the conglomerate particles to carry affinity specific molecules, as described above. Especially preferred Is the use of the particles of the invention for immunoaffinity capture of specific blomacromoleclar entities employing chemical means for coupling the antibody molecules to the surface of the beads. Preferred methods for chemically activating such surfaces comprise conventional methods well-known to a person skilled in the art. Examples of such preferred methods are cyanogenbromide activation, divinylsulfone activation, epichlorohydrine activation, triazine activation, carbodiimide activation, bisepoxyrane activation and activation agents containing at least one of the following functional groups: aldehyde, anhdride, hydrazine, periodate, benzoquinone, tosylate, tresylate, and diazonium groups

Immobilisation of antibodies to activated surfaces is well described elsewhere (see e.g. Harlow & Lane, 1988, Antibodies a Laboratory Manual, Cold Spring Harbor Laboratories) and comprises contacting the antibody molecules at specified conditions of pH, salinity and temperature with chemically activated beads for a specified length of time.

Spacer groups, charged groups or hydrophobic groups can be introduced to achieve adsorbent particles with mixed binding characteristics for "mixed mode" separations.

Affinity capturing and release can be affected as known to the skilled practitioner of conventional affinity chromatography and as described thoroughly elsewhere (see e.g. Affinity Chromatography, Principles and Methods (Pharmacla-LKB), Dean,P.G., et al., eds., 1985, Affinity Chromatography: A practical approach, IRL Press, Oxford, and Scouten, W.H., 1981, Affinity Chromatography, Wiley Intersclence, New York).

The polymeric base matrix is used as a means of covering and keeping multiple core materials together and as a means for binding the active substance. Thus, the polymeric base matrix is to be sought among certain types of natural or synthetic organic polymers, typically selected from
A) natural and synthetic polysaccharides and other carbohydrate based polymers, including agar, alginate, carrageenan, guar gum, gum arabic, gum ghatti, gum tragacanth, karaya gum, locust bean gum, xanthan gum, agaroses, celluloses, pectins, mucins, dextrans, starches, heparins, chitosans, hydroxy starches, hydroxypropyl starches, carboxymethyl starches, hydroxyethyl celluloses, hydroxypropyl celluloses, and carboxymethyl celluloses.
B) synthetic organic polymers and monomers resulting in polymers, including acrylic polymers, polyamides, polyimides, polyesters, polyethers, polymeric vinyl compounds, polyalkenes, and substituted derivatives thereof, as well as copolymers comprising more than one such polymer functionality, and substituted derivatives thereof; and
C) mixture thereof.

A preferred group of polymeric base matrices are polysaccharides such as agarose.

The ideal and preferred shape of a single particle is substantially spherical. The overall shape of the particles is, however, normally not extremely critical, thus, the particles can have other rounded shapes, e.g. ellipsoid, droplet and bean forms, as well as more irregular shapes.

In one preferred embodiment, the particulate material has a density at least 1.3, such as at least 2.0, preferably at least 3.0, more preferably at least 3.5, most preferred at least 4 g/ml, where the particles of the particulate material have an average diameter of 5-75 µm, and the particles of the particulate material are essentially constructed of a polysaccharide base matrix and a core material.

In another embodiment, the particulate material has a density in the range of 6-16 g/ml, where the particles of the particulate material have an average diameter of 10-30 µm, and the particles of the particulate material are essentially constructed of a polysaccharide base matrix and a core material.

In a further embodiment, the particulate material has a density of at least 2.5 g/ml, where the particles of the particulate material have an average diameter of 5-75 µm, and the particles of the particulate material are essentially constructed of a polymeric base matrix selected from polysaccharides, preferably agarose, and a core material, said core material having a density In the range of 6.0-16.0 g/ml where at least 95% of the particles comprises one core material bead having a diameter which is at least 0.70 of the diameter of the particle, said polymeric base matrix including pendant groups which are positively charged at pH 4.0 or which are affinity specific molecules for a bio-molecule.

Alternatively, the adsorbent particles may be in the form of conglomerate particles as disclosed in WO 92/18237 and WO 92/00799 or it may be any other type of particle having the desired characteristics in terms of e.g. size, density, surface chemistry, stability and safety. The particle may be either porous and permeable to the bio-macromolecular entity or substantially non-porous and non-permeable having only the surface area available for binding of the bio-macromolecular entity.

### The method

A column designed for expanded bed adsorption is used in the extracorporeal capture method, e.g. a column with a distribution means at the inlet e.g. in the form of a distribution plate or a localised stirring device as disclosed in WO 92/18237 and WO 92/00799, respectively, the disclosure of which are Incorporated herein by reference. A suitable column can also be the column specifically designed for the All Expanded Process as Illustrated in WO 99/65586 (UpFront Chromatography A/S). Alternatively, the column can be a disposable column likewise illustrated in WO 99/65586, the disclosure of which is incorporated herein by reference. The amount of body fluid, and thereby the amount of adsorbent needed, determines the size of the column needed for the purification.

Prior to contacting the extracellular body fluid with the adsorbent medium the adsorbent is optionally fluidised/expanded in the column with a flow of equilibration buffer that corresponds to 10-1000 cm/h and preferably 200-400 cm/h. The equilibration buffer may consist of 10-100 mM buffer (e.g. Tris, acetate, citrate, glycine, carbonate, phosphate) and typically have a pH value between 7.0-8.0 such as 7.3-7.5, and typically an osmolality similar to 0.9% w/v NaCl. Thus, preferably, the fluidised bed of the particulate material is washed with an equilibration buffer prior to contacting with the body fluid. The adsorbent is most conveniently equilibrated in the column and the equilibration buffer is preferably applied at least until the bed is stabilised.

After the stabilisation of the bed, the body fluid is contacted with the expanded bed with a flow rate typically in the range of 0.1-100 cm/min, such as 1-20 cm/min, preferably at 1-12 cm/min, more preferably at approximately 2-10 cm/min, such as 2-8 cm/min, preferably 3-6 cm/min. These rates depend on the type of extracellular body fluid and the matrix used.

It is recommended that the following expansion ratios (H/H₀) is 1 - 5 times, advantageously 1.2 - 3 times, however 1.5 - 2 times is usually recommended. By expansion ratio is meant the relation between the volumes of the fluidised matrix and the sedimented matrix.

Another parameter of importance is the amount of particulate material (adsorbent) used to capture certain amount of the bio-macromolecular entity. Typically, the ratio between the bio-macromolecular entity and the particulate material (adsorbent) is in the range of 0.1-100.0 mg bio-macromolecular entity/ml adsorbent, preferably at least 0.50 mg/ml, more preferably at least 2 mg/ml, still more preferable at least 5 mg/ml and most preferable at least 10 mg/ml.

### Magnetically stabilised fluidised bed

In another embodiment of the invention the stabilised fluidised bed is comprised by magnetically stabilised fluidised bed wherein the adsorbent medium is specifically designed for use in a magnetically stabilised fluidised bed.

In one embodiment, the magnetizable adsorbent medium is comprised by porous, ion-exchange particles prepared from agarose based ion-exchange particles which have been treated with a ferrofluld solution see, e.g. US 5,084,169.

In another embodiment, the magnetizable adsorbent medium is a homogeneous composition of an anion exchange resin, a soft ferromagnetic substance, and a water permeable organic polymer binder as described in US 5,230,805 which is incorporated herein by reference.

In a preferred embodiment the magnetizable adsorbent medium comprises one or more pendant groups, as described above, which are affinity specific molecules for one or more bio-macromolecular entities.

### Stirred tank

In an important embodiment of the invention, advantage is take by the unique features of an adsorbent medium which comprises very dense particles. To a large extent, the use of such particles avoid the important problem of adsorbent medium particle carry-over from the continuous stirred incubation container.

Said adsorbent medium is typically in the form of particles having a density of at least 1.3 g/ml and a mean diameter in the range of 5-1000 µm, such as a density of at least 1.5 g/ml and a mean diameter in the range of 5-300 µm, preferably a density of at least 1.8 g/ml and a mean diameter in the range of 5-150 µm, and most preferred a density of more than 2.5 g/ml and a mean diameter in the range of 5-75 µm.

As described previously, a very important feature of the particulate material is the fact that the polymeric base matrix comprises either chargable pendant groups or affinity specific molecules for a bio-macromolecular entity as pendant groups. Said pendant groups have been described In details above and is referred to here.

### Applications

The invention disclosed is suitable for extracorporeal removal of specific cells, microoganisms, or particulate matters from body fluids. It may be of potential value in a diverse range of conditions (infections, malignancies, and autoimmune diseases) and maybe also In a negative selection scheme for preparation of higly specific cell subsets in transfusion or transplantation medicine.

One application for extracorporeal specific cellular depletion (ECD) could be to remove virusinfected cells from the blood. Virus infected cells in the blood occur in a number of conditions. Since these cells express virally encoded proteins on their surface they are amenable for specific targeting e.g. by antibodies. This is what the immune system is trying to do anyway and it is possible that even a partial removal of virus-loaded cells will ease the job of the immune system. It is also possible that infections associated with immune defects (e.g. AIDS) will be temporarily aggravated by removal of virus infected immune cells but the treatment should still decrease the infectious load and therefore help the body restoring levels of non-infected cells.

### Examples of serious infections with virus in blood cells are:

HIV (AIDS) - HIV infects CD4-positive T-lymphocytes and a minor fraction of these infected cells are circulating. HIV is frequently mutating and so is a difficult target for antibody therapy but constant epitopes may be expressed by infected cells. The largest reservoir of HIV, however, is found outside the circulation in cells in lymphoid tissues.

Parasites. Malaria parasites spend some of their time in red blood cells and if these express specific markers they could be removed by ECD and help treating the condition. Resistance to drugs is an increasing problem in malaria treatment.

Another important application would be to remove cancer cells from the blood. Leukemias (blood cancers), especially lymphocytic and myeloid leukemias and myelomatosis may benefit from specific cell removal as an alternative or adjunct to cytotoxic drugs (that are non-specific). If the abnormal cells express specific surface markers (and many malignant cells do) their selective removal by ECD is possible. Occassionally, leukopheresis (extracorporeal selective removal of leukocytes based on centrifugation methods) is already used for the treatment of some leukemias which argues for the benefits of ECD in this area.

It is possible to remove apoptotic bodies (which present a specific antigens - e.g. phosphatidylserine - on the cell surface) and other cellular debris from the blood. In some immune diseases (e.g. systemic lupus erythematosus) it appears that the normal mechanisms for removal of dying cells and cell residues are impaired and these components therefore accumulate in the blood. Their removal, e.g. by phosphatidylserine-specific receptors would probably alleviate these autoimmune diseases.

Also removal of specifically reacting cells seem interesting. In autoimmune disorders tissue and organ damage are caused by self-reactive immune cells. These cells (when circulating) could be removed by ECD using the antigens that they react with as receptor molecules. A few examples (with targets in parentheses) are rheumatoid arthritis (collagen), diabetes type I (islet cell antigens), Sjögren's disease (exocrine glands), systemic lupus erythematosus (double-stranded DNA), myasthenia gravis (acetylcholine receptor). For some of these diseases (diabetes, Systematisk Lupus Erythmatosus (SLE), myasthenia gravis and others) there are animal models in which the principle could be tested.

Preparation of specific cellular fractions for transfusion may also be conceived. Blood banks already fractionate the components of blood (centrifugation methods) to minimise the volume infused (minimises infection risks) and to use the blood donations rationally. ECD-methods would make it possible to extend this strategy by removing all but the specific cells that the patient needs, e.g. specific subsets of lymphocytes or enrichment of stem cells in a negative selection scheme. Also, it may be possible to remove damaged/infected cells by ECD before transfusion. This can be achieved by employing broadly reacting receptor molecules as ligands.

And finally, ECD-methods can be used for the specific capture of rare bio-macromolecular entities for diagnostic purposes. One example is the capturing of prions for diagnosis of encephalopatic conditions. Another example is the capturing of fetal cells in the mother blood with the Intention of early diagnosis of inheritable diseases.

### Description of the Figures

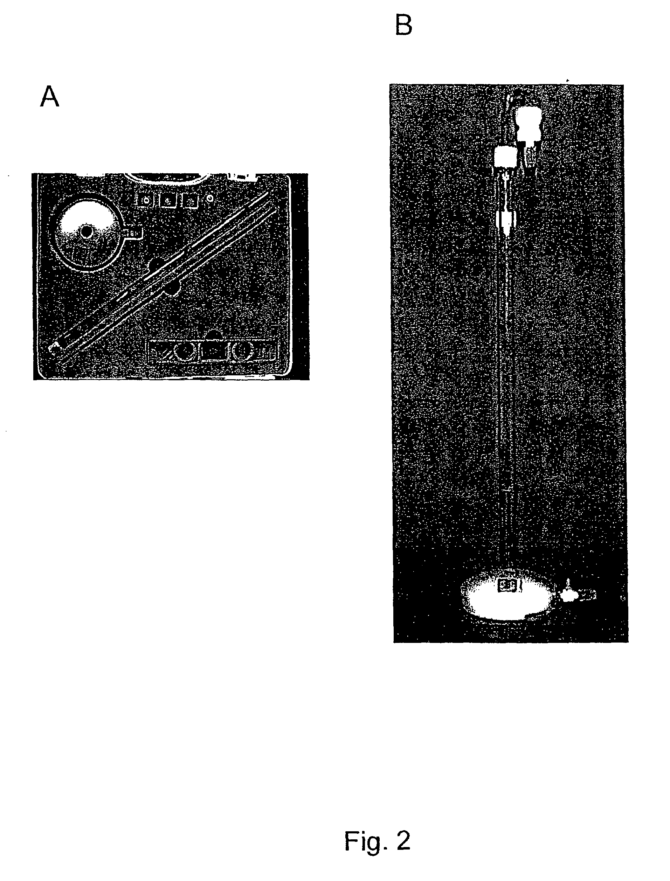

FIGURE 1. Principle of continuous extracorporeal adsorption.
   Shown In the figure is a vessel "a" continuously receiving blood from the patient and connected to a stabilised fluidised bed column ("b") through a valve which may be closed or open. The blood stream is applied in an upward direction from the bottom of the stabilised fluidised bed and is then led from the top of the column through another valve to vessel "c" which continuously delivers blood back into the patient. The "valves" may be in the form of pump for continuous of intermittent distribution of blood to the column, or a separate pump (not shown) may be utilised
FIGURE 2. Upfront Chromatography column (7010-0000).
   The figure Illustrates the set-up of an stablised fluidised bed column using commercially available equipment (Upfront Chromatography A/S, 7010-0000). The equipment comprises a vertical glass column held in place by a foot plate also containing tube connector for the inlet fluid. Furthermore the glass column is equipped with an outlet fluid connector at the top of the column. (A) Shows the column disassembled in its transport container. (B) Shows the assembled column.

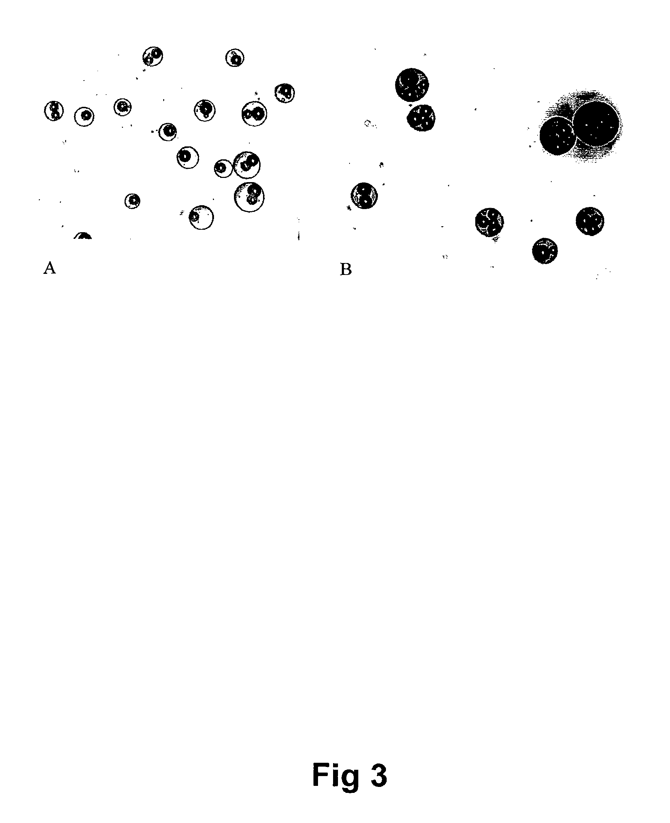

FIGURE 3. Biotin-coupled conglomerate beads with a core of glass particles stained by DAB (+blotin) and nonstained (-biotin)
   Conglomerate adsorbent particles with cores of glass particles, either underivatised (A) or derivatised with biotin as the ligand (B). Both types of particles were used for stabilised fluidised bed chromatography of EDTA-stabilised human blood spiked with avidin-peroxidase as described in example 2. After chromatography and wash a sample of each type of adsorbent beads were subjected to DAB-staining to reveal the presence of peroxidase activity on the surface of the beads. Peroxidase activity gives rise to a brown (dark In the black & white figure) coloration of the beads as seen for the blotin-coupled beads but not for the non-derivatised beads.

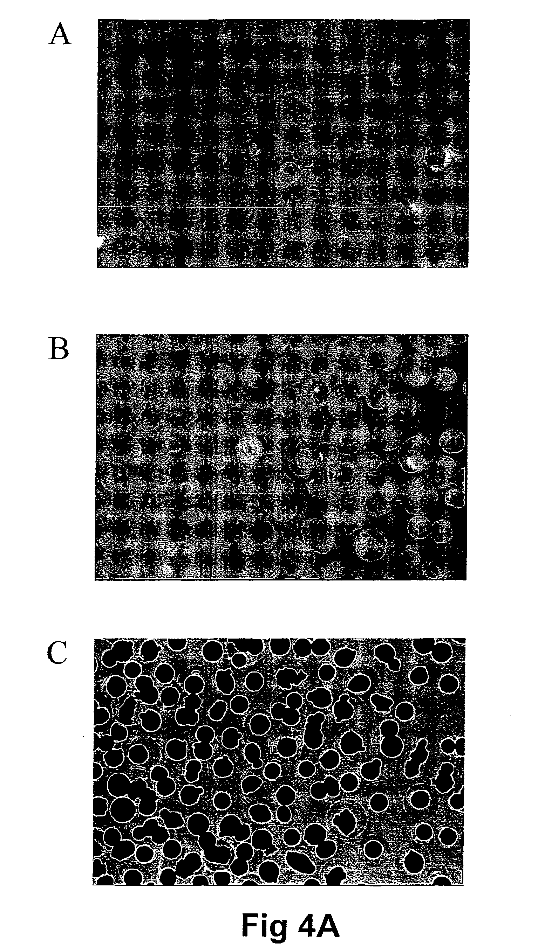

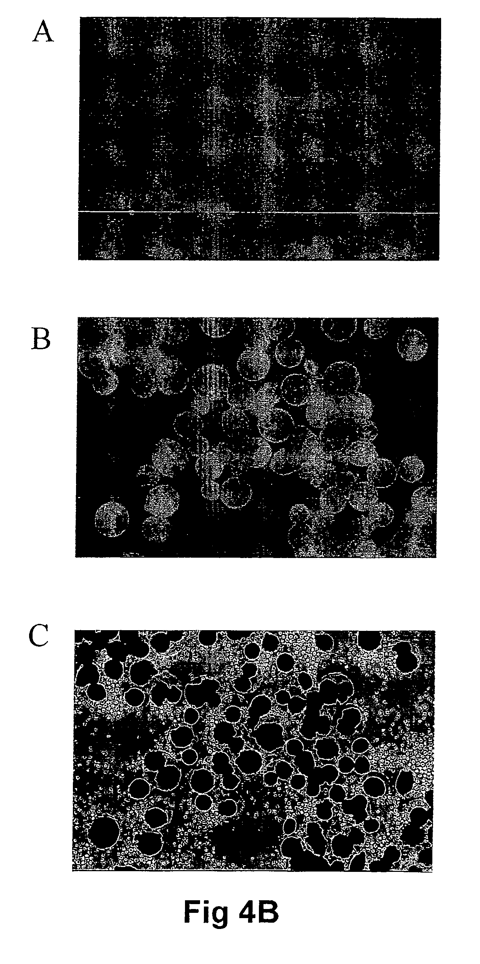

FIGURE 4a and b. Z109-coupled beads (agarose with stainless steel particles) binding Cy-3-labelled mouse Mab (with and without blood).
   Figures 4a and 4b show conglomerate adsorbent particles with cores of stainless steel particles either underivatised (A) or coupled with rabbit anti mouse immunoglobulin antibodies at 3 mg/ml (B and C). The beads were contacted with a Cy3-labeled purified monoclonal mouse antibody either in PBS (Figure 4a) or spiked to whole heparinized bovine blood (Figure 4b) in a batch incubation followed by wash in PBS. Beads were inspected for fluorescence at 570 nm (A+B) and also in normal light (C). The presence of Cy3-fluorescent molecules in the beads is revealed by a bright red emission as is seen for the antibody-coupled beads but to a much lesser degree for the non-derivatised beads with the Cy3-immunoglobulin in PBS as well as in blood.

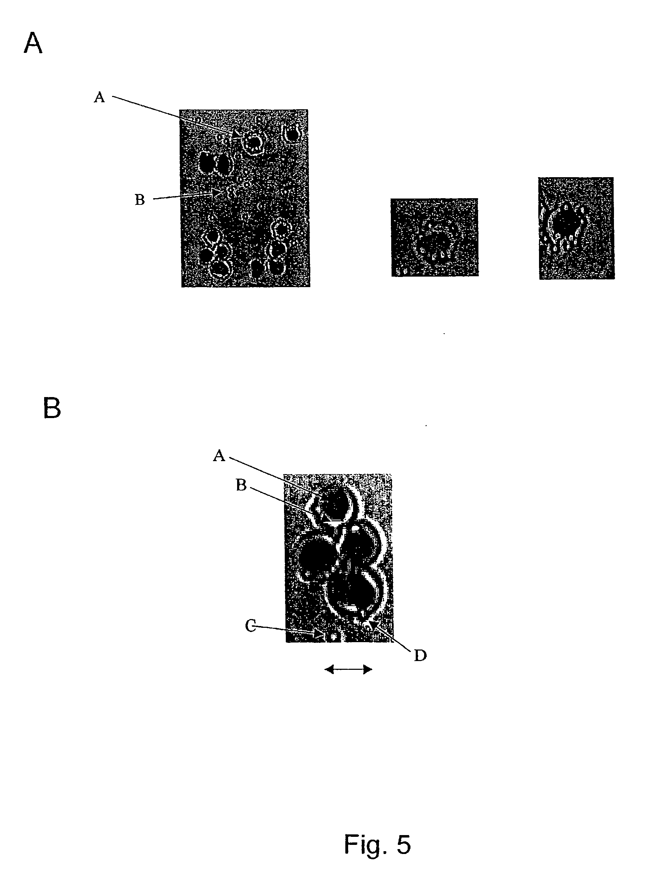

FIGURE 5. PEI-agarose beads with a core of stainless steel particles, binding of blood cells.
   Figure 5A shows that after incubation of whole EDTA-stabilised human blood in a batch procedure with stainless steel/agarose-PEI beads some of the blood cells bind to the outer surface of the beads (A) while others are not bound (B). On closer inspection (Figure 5B, double arrow represents approximately 25 micrometer) the stainles steel core particle (A), the agarose coating layer (B) and unbound (C) as well as bound blood cells (D) are seen.

### Examples

### Example 1.

**The basics of a stabilised fluidised bed procedure.**

### A. Running human whole blood through a stabilised fluidised bed (EDTA-stabilised blood)

The purpose of the following example is to demonstrate the feasibility of running human non-separated blood through a stabilised fluid bed of high density, low diameter adsorbent beads.

### Materials and methods:

The experimental fluidised bed column set-up was established based on the following standard laboratory equipment:
- Pump (Ole Dich Aps, Denmark)
- Silicone tubing (MasterFlex)
- Magnetic stirrer (Janke and Kunkel)
- Column: UpFront Chromatography A/S, Denmark (cat.no. 7010-0000), see figure 2 which also shows the assembled equipment.

### Adsorbent beads (without ligand):

Test-beads were kindly provided by UpFront Chromatography A/S, Denmark. The beads had the following characteristics:
- Bead composition: epichlorohydrin cross-linked agarose (4 % w/v) with a core of tungsten carbide
- Bead shape: Mainly spherical.
- Diameter: 20-40 □m.
- Average Individual bead density in the hydrated state: 4.1 g/ml.
- Void volume in sedimented state: Approx. 40 % of packed volume
- Theoretical bead surface area per litre sedimented beads: Approx. 120 m2
(theoretical surface area was calculated from estimating that 1 litre sedimented bed corresponds to 600 ml beads (the non-void volume), each having a volume of 14130 µm³ from which the number of beads could be calculated to be 42x10⁹. With each bead having an outer surface area of 2826 µm² this gives a total bed surface area of 120 m² for 1 litre of beads).

### Adsorbent equilibration buffer:

6 % w/v dextran MW 110.000 (Pharmacosmos, Denmark) in 0.9 % w/v sodium chloride was used to pre-equilibrate the adsorbent before percolation of the blood through the column.

### Blood:

A freshly drawn human blood sample from a healthy donor, collected in standard EDTA glass tubes (Becton Dickinson, code no. 15067), was used for the experiment. The blood was stored at room temperature and used within 1 hour after collection.

### Procedure:

The fluid bed column (diameter: 1 cm) was assembled according to the supplier's instructions and added to an aqueous suspension of the adsorbent beads to reach a sedimented bed height of 7 cm (5.5 ml, corresponding to approx. 0.7 m² bead surface area). Then an upward flow of the adsorbent equilibration buffer of approx. 5 ml/min was applied in order to fluidise and wash the beads with the buffer and in order to ensure an optimal salt concentration/osmolality for minimal hemolysis of the blood cells when entering the column.

The column was adjusted to a completely vertical position in order to secure an even flow inside the column.

When the beads were fluidised (i.e. when the fluidised bed height reached above 10 cm) the magnetic stirrer at the bottom of the column was engaged at approx. 80 % full speed in order to ensure an even distribution of the incoming liquid and the flow rate was adjusted to 2.2 ml/min. The washing with adsorbent equilibration buffer was continued for 15 min. in which time a stabilised fluidised bed was formed with a fluidised bed height of 16 cm. The stability of the fluidised bed was ascertained by a careful visual inspection of the bed and the beads inside the column using a magnifying glass as a visualisation aid. The lack of visual channelling and back mixing was taken as an indication of the bed stability.

Following the establishment of a stabilised and equilibrated fluid bed, 100 ml human blood was pumped into the column at a steady flow rate of 2.2 ml/min.

### Results:

The entry and penetration of the blood Into the stabilised fluidised bed was carefully followed by visual inspection: A well-defined weakly parabolic front of the red blood sample was moving at constant speed through the stabilised fluidised bed and no back-mixing and channelling was observed anywhere in the entire stabilised fluidised bed. When the blood sample occupied the entire volume of the stabilised fluidised bed the height of the bed had increased to 21 cm (i.e. 3 times the sedimented bed height). Although the non-transparency of the blood sample made it very difficult to observe the adsorbent beads inside the column, the use of a magnifying glass made it possible to ascertain the bed height as well as the absence of channelling in the bed.

Following the break-through of the blood sample at the top of the stabilised fluidised bed, the run through was collected in fractions of 5 ml blood while continuing the application of the full 100 ml blood sample to the column. The collected fractions were centrifuged at 500 G for 10 min and the degree of hemolysis occurring after passage of the sample through the column was determined by spectrophotometry at 540 nm using an untreated blood sample as a reference sample. All collected fractions had a degree of hemolysis below 2 % of the total number of erythrocytes (as determined in a fully experimentally hemolysed control sample). Further, microscopic examination of the collected blood samples did not reveal any occurrence of clotting of the blood and neither could any adsorbent beads be detected in the samples.

Following the application of the 100 ml blood sample, the column was percolated with the adsorbent equilibration buffer again with the aim of washing out the remaining blood inside the column. The washing was also conducted at a flow rate of 2.2 ml/min. The entry of the buffer and the gradual washing of the column gave rise to a sharp and upwardly moving boundary between the incoming equilibration buffer and the blood sample, indicating a stable fluidisation of the bed, devoid of channelling and back mixing, with fluids moving in plug flow. When the blood sample had been completely washed out of the column, the fluidised bed height had returned to 16 cm.

Following the washing of the stabilised fluidised bed with equilibration buffer a sample of the beads from inside the column was microscopically examined. It was observed that all beads were fully intact with smooth surfaces and no cells adhering to them.

In this experiment an EDTA-stabilised, human full blood sample of 100 ml was applied to and pumped through a stabilised fluidised bed with a sedimented bed height of 7 cm (5.5 ml). The overall conclusion is that although it could be expected to give rise to significant problems to apply full blood comprising 40-50 % by volume of blood cells as well as a high concentration of proteins to a stabilised fluidised bed of small adsorbent beads in the form of the break-down of the stabilised fluid bed system and/or significant negative effects on the blood, no serious problems were observed during the entire procedure. Furthermore, beads could be washed completely free of blood and the entire bed was reversed to its initial state after passage of the blood and subsequent washing.

### B. Running human whole blood through a stabilised fluidised bed (heparin-stabilised blood)

The same experiment as in example 1A was performed with the only exception that heparinized human blood was used instead of EDTA stabilised blood. The blood for this experiment was collected in standard heparin glass tubes (Venoject, NaHeparin, Terumo Europe).

The results obtained were similar to the results described above for EDTA-stabilised human blood and thus indicate that the stabilised fluidised bed procedure can be performed without problems with heparinized human blood as well as with EDTA stabilised blood.

### Example 2.

### Specific adsorption of a biomacromolecular entity from whole human blood in a stabilised fluidised bed; binding of avidin-peroxidase by biotin-coupled beads.

The aim of the following experiment was to establish the feasibility of binding of a specific bio-macromolecular entity from whole human blood in a stabilised fluidised bed procedure. For the sole purpose of demonstrating such binding an enzyme-conjugate was used as a model protein as this allowed a sensitive assay to be performed in order to demonstrate the binding of the enzyme. The test substance (peroxidase-labelled avidin) was added to whole human blood followed by adsorption of the test substance to a high-density biotin labelled adsorbent In a stabilised fluidised bed procedure. Binding of the test substance to the adsorbent was then demonstrated by the development of staining on the adsorbent beads though the action of the bound peroxidase conjugate using a suitable indicator enzyme substrate (diaminobenzidine).

This example supplements example 1 in showing the feasibility of using another type of beads with a lower density and bigger diameter and with a core of glass particles for stabilised fluidised bed chromatography of whole blood.

### Materials and methods:

The experimental set-up and equipment used was the same as in example 1.

### Adsorbent beads (with biotin as ligand):

The adsorbent used for this experiment was a high-density biotin-agarose/glass adsorbent (product no.: 6302-0000, UpFront Chromatography A/S, Denmark). This adsorbent has the following characteristics:
- Bead composition: epichlorohydrin cross-linked agarose (6 % w/v) with a core of spherical glass particles (See also fig. 3).
- Bead shape: Mainly spherical.
- Diameter: 100-300 µm.
- Average individual bead density in the hydrated state: 1.5 g/ml.
- Ligand: Biotin.

### Adsorbent equilibration buffer:

6 % w/v dextran MW 110.000 (Pharmacosmos, Denmark) in 0.9 % w/v sodium chloride was used to pre-equilibrate the adsorbent before percolation of the blood through the column.

### Blood:

A freshly drawn human blood sample from a healthy donor, collected in standard EDTA glass tubes (Becton Dickinson, code no. 15067), was used for the experiment. The blood was stored at room temperature and used within 1 hour after collection. Just prior to the adsorption procedure, 1 ml horseradish peroxidase labelled avidin (avidin-peroxidase, 1 mg/ml, Product no.: 4030Y, Kem-En-Tec A/S, Denmark) was added to 100 ml of the blood to give a final concentration of 10 microgram avidin-peroxidase per ml human blood.

### Procedure:

The fluid bed column was assembled according to the supplier's instructions and added an aqueous suspension of the adsorbent beads to reach a sedimented bed height of 5.8 cm.

In order to ensure an optimal salt concentration/osmolality for minimal hemolysis of the blood cells when entering the column, a wash with adsorbent equilibration buffer was initially performed at a flow rate of 2.2 ml/min. When the adsorbent beads were fluidised by the upward flow of equilibration buffer the magnetic stirrer was engaged at 80 % full speed and the column was positioned carefully to a completely vertical state. When reaching a fully stabilised fluidised state, the height of the adsorbent bed had increased to 10.5 cm.

Following the initial wash with equilibration buffer, the blood sample was applied to the column with a flow rate of 2.2 ml/min. A well-defined weakly parabolic front of blood was then observed moving gradually up through the stabilised fluidised bed. No back mixing or channelling was observed throughout the experiment. When fully loaded with the human blood, the bed height was determined using a magnifying glass to be approx. 14.5 cm (i.e. 2.5 times the sedimented bed height).

The degree of hemolysis of the blood having passed the column was determined by spectrophotometry at 540 nm (as in example 1) to be below 0.2 %.

Following the application of the blood sample the column was washed with 200 ml adsorbent equilibration buffer in order to wash out the blood and any unbound peroxidase labelled avidin.

After washing the stabilised fluidised bed thoroughly, a sample of the adsorbent beads was incubated for 2 minutes with diaminobenzidine substrate (cat. no.: 4150, Kem-En-Tec A/S, Denmark) prepared according to the suppliers instructions.

### Results:

The diaminobenzidine enzyme substrate gave a very strong brown colouring of the adsorbent beads thus demonstrating the presence on the beads of bound avidin-peroxidase extracted from the blood during the passage of the blood through the stabilised fluidised bed (see fig. 3).

An experiment performed with the same type of adsorbent beads but lacking the biotin ligand gave a negative result in this enzyme substrate test thus indicating that the first result was due to a specific interaction and binding between the biotin ligand on the adsorbent beads and the avidin-peroxidase added as a test substance to the human blood sample (see fig. 3)

### Example 3.

### Specific adsorption of a biomacromolecular entity from whole bovine blood in a batch operation, binding of mouse immunoglobulin by anti-mouse antibody-coupled beads.

The aim of this example was to demonstrate the feasibility of using an anti-mouse immunoglobulin antibody-coupled adsorbent for the extraction of mouse antibodies added to whole bovine blood.

The adsorbent used for this experiment was a high density divinylsulfone-coupled agarose/stainless steel adsorbent (Upfront Chromatography A/S, Denmark) to which an anti-mouse immunoglobulin antibody from rabbits (code no. Z0109, DAKO A/S, Denmark) was coupled.
- Bead composition: epichlorohydrin cross-linked agarose (4 % w/v) with a core of stainless steel particles (fig. 5).
- Bead shape: Mainly spherical.
- Diameter: 20-40 µm.
- Average individual bead density in the hydrated state: 3.8 g/ml.
- Ligand: Rabbit anti-mouse immunoglobulin (DAKO A/S, Denmark, code no. Z0109) coupled through divinylsulfone at 3 mg/ml

### Procedure:

Freshly obtained heparinized whole bovine blood was obtained from a healthy donor collecting the blood in heparin-tubes (Venoject, NaHeparin, Terumo Europe). For the purpose of demonstrating the selective extraction of mouse immunoglobulin from the blood, 100 µl Cy3-labelled mouse immunoglobulin prepared from a kit obtained from Amersham Pharmacia Biotech (code no. PA33000) and according to the manufacturers instructions was added to 0.5 ml bovine blood and incubated (slow rotation) with a 100 µl suspension of adsorbent beads for 30 minutes at room temperature. The mouse immunoglobulin was protein A-purified, IgG1 isotype and used at 3.7 mg/ml in PBS. In parallel with this a similar incubation was performed with 100 µl Cy3-mouse immunoglobulin in PBS (no blood, positive control) and both of these incubations were also performed with non-coupled beads (negative control).

The adsorbent beads were washed prior to use by incubation and decanting with PBS (2 times) prior to the incubation with the blood/mouse antibody mixture. After 30 minutes of incubation, beads were retrieved by decantation, washed two times with PBS(incubation/decantation) and then inspected by visual and fluorescence microscopy (at 570 nm).

### Results:

As seen in figure 4, the Z0109-derivatized beads bound the Cy-3-labelled mouse antibody both when supplied In pure solution (PBS) and spiked into whole heparinized blood at a 5 times lower concentration while a very low background binding was observed with non-derivatised beads. As the intensity of the fluorescence of the beads were similar when binding was performed with the pure Cy3-immunoglobulin solution as compared to when the incubation was performed with Cy3-immunoglobulin spiked to whole blood the binding of the immunoglobulin to the beads were clearly not affected by the presence of whole blood. Fluorescence was confined to the outer surface of the polymeric base matrix (the agarose layer) as would be expected (see figure 4).

In conclusion this experiment shows the feasibility of using small adsorbent beads for batch-wise specific retrieval of a soluble blo-macromolecular entity (labelled immunoglobulin molecules) from whole bovine blood.

### Example 4.

### Adsorption of blood cells to a high density adsorbent in a continuous stirred tank reactor using polyethyleneimine-coupled beads

The aim of the following example was to demonstrate the feasibility of binding human blood cells to a high-density ion-exchange adsorbent in a stirred tank reactor.

The adsorbent used for this experiment was a high-density polyethyleneimine (PEI) agarose/stainless steel adsorbent (UpFront Chromatography A/S, Denmark). This adsorbent has the following characteristics:
- Bead composition: epichlorohydrin cross-linked agarose (4 % w/v) with a core of stainless steel particles (See also fig. 5).
- Bead shape: Mainly spherical.
- Diameter: 20-40 µm.
- Average individual bead density in the hydrated state: 3.8 g/ml.
- Ligand: polyethyleneimine (PEI)

Whole EDTA-stabilised human blood (100 ml) obtained as described in example 1 was mixed with 1 ml adsorbent beads for 10 minutes under careful agitation at room temperature. Following sedimentation of the adsorbent beads, the blood sample was decanted and the beads were washed with adsorbent equilibration buffer (incubating and decanting the buffer) followed by microscopic examination.

As illustrated in Figure 5 the adsorbent binds the blood cells to its surface adsorbent polymeric matrix layer.

### Example 5.

### Specific binding of cells in a cell suspension directly to antibody-coated high density conglomerate particles in a batch process.

The purpose of this example is to demonstrate that antibody-coated conglomerate adsorbent beads can be used for immuno-affinity chromatography of whole cells.

The adsorbent used for this experiment is an divinylsulfone-activated (low activation level) agarose/stainless steel adsorbent (Upfront Chromatography A/S, Denmark) to which a mouse anti-bovine CD8 antibody (monoclonal, IgG1, ATCC CLR1871) is coupled:
- Bead composition: epichlorohydrin cross-linked agarose (4 % w/v) with a core of stainless steel particles (See also fig. 5).
- Bead shape: Mainly spherical.
- Diameter: 20-40 µm.
- Average individual bead density in the hydrated state: 3.8 g/ml.
- Ligand: Monoclonal mouse anti-bovine CD8 immunoglobulin (ATCC CLR1871) coupled through divinylsulfone

### Procedure:

Freshly obtained heparinized whole bovine blood is obtained from a healthy donor by collecting the blood in heparin-tubes (Venoject, NaHeparin, Terumo Europe). Peripheral blood mononuclear cells (PBMCs) are prepared by standard methods (density gradient centrifugation through Ficoll^{™}, (Amersham Pharmacia Biotech, code no. 17-1440-03) according to standard procedures (Rickwood ed., 1984, Centrifugation: a practical approach, IRL Press) and resuspended in PBS at approximately 10⁶ PBMCs pr ml. This cell suspension is prepared from fresh blood and it is used immediately after preparation.

As a model experiment solely to demonstrate the ability of the adsorbent beads to bind whole cells efficiently, 500 µl of the PBMC suspension is first mixed with 100 µl Cy-3-conjugated antibody against bovine CD8 (the same antibody used for coupling to the beads) (3.7 mg/ml) prepared using a Cy3 labelling kit from Amersham Pharmacia Biotech (code no. PA 33000) according to the instructions supplied with the kit. After 30 minutes at room temperature this mixture is then incubated with 200 µl antibody-coupled beads in PBS. As a negative control, non-derivatised beads of a similar composition are also incubated in a separate experiment. The adsorbent beads are washed prior to incubations by incubation and decanting with PBS (3 times). After 30 minutes of gentle agitation at room temperature followed by 3 times wash in PBS, the resulting suspension is investigated in a fluorescence microscope in visual light and at 570 nm to reveal the presence and localisation of CD8-positive (Cy3-labelled) cells In the suspension.

### Results:

It is to be expected from this experiment that the antibody-derivatised beads clearly show up under the microscope with the smaller CD-8-positive (and therefore Cy-3-fluorescent) PBMCs attached to the surface of the beads with none or very few non-fluorescent cells attached. This pattern is clearly different from the random pattern of fluorescent (CD8-positive) and non-fluorescent cells seen with non-derivatised conglomerate particles, further demonstrating the specific nature of the binding of cells to the immunoadsorbent particles.

### Example 6.

### Specific binding of cells in a cell suspension indirectly to antibody-coated high-density conglomerate particles by means of a catching antibody in a batch process.

The purpose of this example is to demonstrate that antibody-coated conglomerate adsorbent beads can be used for indirect immuno-affinity chromatography of whole cells.

The adsorbent used for this experiment is a divinylsulfone-activated (low activation level) agarose/stainless steel adsorbent (Upfront Chromatography A/S, Denmark) to which a rabbit anti mouse immunoglobulin (DAKO code no. Z0109) is coupled:
- Bead composition: epichlorohydrin cross-linked agarose (4 % w/v) with a core of stainless steel particles (See also fig. 5).
- Bead shape: Mainly spherical.
- Diameter: 20-40 µm.
- Average Individual bead density in the hydrated state: 3.8 g/ml.
- Ligand: Rabbit anti mouse immunoglobulin (DAKO Z0109 coupled through divinylsulfone

### Procedure:

Freshly obtained heparinized whole bovine blood is obtained from a healthy donor by collecting the blood in heparin-tubes (Venoject, NaHeparin, Terumo Europe). Peripheral blood mononuclear cells (PBMCs) are prepared by standard methods (centrifugation through Ficoll^{™}, (Amersham Pharmacia Biotech, code no. 17-1440-03) according to standard procedures (Rickwood ed., 1984, Centrifugation: a practical approach, IRL Press) and resuspended in PBS at approximately 10⁶ PBMCs pr ml. This cell suspension is prepared from fresh blood and it is used immediately after preparation.

As a model experiment solely to demonstrate the ability of the adsorbent beads to bind whole cells efficiently, 500 µl of the PBMC suspension is first mixed with 100 µl Cy-3-conjugated antibody against bovine CD8 (3.7 mg/ml) prepared using a Cy-3 labelling kit from Amersham Pharmacia Biotech (PA 33000) according to the instructions supplied with the kit. After 30 minutes at room temperature this mixture is then washed carefully 3 times to remove surplus of Cy3-labelled antibody and then incubated with 200 µl Z0109-coupled beads in PBS. As a negative control, non-derivatised beads of a similar composition are also incubated in a separate experiment. The adsorbent beads are washed by incubation and decanting with PBS (3 times) prior to the incubation with the PBMC. After 30 minutes of gentle agitation at room temperature followed by 3 times wash in PBS the resulting suspension is investigated in a fluorescence microscope in visual light and at 570 nm to reveal the presence and localisation of CD8-positive (Cy3-labelled) cells in the suspension.

### Results:

It is to be expected from this experiment that the antibody-derivatised beads clearly show up under the microscope with the smaller CD-8-positive (and therefore Cy-3-fluorescent) PBMCs attached to the surface and none or very few non-fluorescent cells attached. This pattern would clearly be different from the random pattern of fluorescent (CD8-positive) and non-fluorescent cells seen with non-derivatised conglomerate particles, and would demonstrate the specific nature of the binding of cells to the immunoadsorbent particles.

Thus this experiment is designed to show that it is possible to prepare a "universal" immunoadsorbent for monoclonal antibodies of any kind, using a polyclonal mouse immunoglobulin-specific antibody as a ligand coupled to the adsorbent conglomerate particles. This is an advantage, as some monoclonal antibodies as know to a person skilled in the art will not function after covalent (chemical) immobilisation to solid surfaces. Furthermore this will allow the use of a stabilised fluldised bed generated from such general immunoadsorbent particles in a device for catching other antibodies, e.g. after the reaction In the solution phase of these second antibodies with constituents in body fluids ("bind-and-catch" approach).

### Example 7.

### The use of a stabilised fluidised bed comprising immunoadsorbent high density conglomerate particles for the removal of CD8-positive T-cells in a live host.

To demonstrate the feasibility of using a stabilised fluidised bed for the extracorporeal specific removal of a T-cell subset from the blood-stream of a cow, high density adsorbent particles are derivatised with an antibody against cow CD8 by coupling a monoclonal mouse antibody against bovine CD8 (ATCC CLR1871) through divinylsulfone to the beads.

### Adsorbent beads (without ligand):

Test-beads are provided by UpFront Chromatography A/S, Denmark. The beads have the following characteristics:
Bead composition: epichlorohydrin cross-linked agarose (4 % w/v) with a core of tungsten carbide
Bead shape: Mainly spherical.
Diameter: 20-40 µm.
Average individual bead density in the hydrated state: 4.1 g/ml.
Void volume in sedimented state: Approx. 40 % of packed volume.
Theoretical bead surface area per litre sedimented beads: Approx. 120 m²

### Adsorbent equilibration buffer:

6 % w/v dextran MW 110.000 (Pharmacosmos, Denmark) in 0.9 % w/v sodium chloride is used to pre-equilibrate the adsorbent before percolation of the blood through the column.

### Procedure:

The fluid bed column (diameter: 1 cm) is assembled according to the supplier's instructions and added an aqueous suspension of the adsorbent beads to reach a sedimented bed height of 7 cm (5.5 ml, corresponding to approx. 0.7 m² bead surface area). Then an upward flow of the adsorbent equilibration buffer of approx. 5 ml/min is applied in order to fluidise and wash the beads with the buffer and in order to ensure an optimal salt concentration/osmolality for minimal hemolysis of the blood cells when entering the column. The column is adjusted to a completely vertical position in order to secure an even flow inside the column. When the beads are fluidised (i.e. when the fluidised bed height reached above 10 cm), the magnetic stirrer at the bottom of the column is engaged at approx. 80 % full speed in order to ensure an even distribution of the incoming liquid and the flow rate is adjusted to 2.2 ml/min. The washing with adsorbent equilibration buffer is continued for 15 min. in which time a stabilised fluidised bed is formed with a fluidised bed height of 16 cm. The stability of the fluidised bed is established by a careful visual inspection of the bed. Following the establishment of a stabilised and equilibrated fluid bed, 300 ml bovine blood is pumped Into the column at a steady flow rate of 2.2 ml/min. This is achieved by connecting the tubing through a syringe to a suitable vein in a cow and pumping blood in a continuous process through the column from the bottom inlet and returned to another suitable vein in cow from the top outlet. Small samples of blood are taken from the top outlet each 5 minutes throughout the experiment. The adsorption is run for 1 hour at 5 ml per minute and then terminated. Clotting is avoided by continuously adding a heparin solution in PBS amounting to 25 IU/ml blood through a valve at the bottom inlet of the column.

The results will show if the whole operation can be performed without the occurrence of clotting of the blood, without any extensive cell damage and with no harm to the animal. Furthermore, analysis of the collected outlet-fractions by flow cytometry will demonstrate the extent of which CD8-cells are depleted from the outlet blood stream.

### Example 8.

### Bind and catch example: The use of a stabilised fluidised bed comprising immunoadsorbent high density conglomerate particles derivatised with anti immunogloblin for the removal of CD8 positive T-cells in a live host.

The purpose and execution of this example is similar to example 11, except that a CD8-specific antibody is first injected intravenously into a cow as a bolus injection of 20 ml sterile PBS containing 1 mg/ml mouse anti-CD8. This is then followed by extracorporeal adsorption as described in example 11 to adsorbent particles having anti-mouse immunoglobulin (DAKO Z0109, 3 mg/ml) as attached ligand.

The results will show if the whole operation can be performed without the occurrence of clotting of the blood, without any extensive cell damage and with no harm to the animal. Furthermore, analysis of the collected outlet-fractions by flow cytometry will demonstrate if CD8-cells are depleted from the outlet blood stream with a capacity depending on the volumen of the bed.

The present invention may be further described and defined in the following items:
1. A device for continuous extracorporeal capturing of one or more specific bio-macromolecular entities from an extracellular body fluid obtained from a mammal, said device comprising (i) an inlet tube for obtaining the mammalian extracellular body fluid, (ii) a stabilised fluidised bed comprising adsorption particles characterised by having a specific affinity for one or more specific bio-macromolecular entities and (iii) an outlet tube for reinfusing the treated extracellular body fluid into the mammal from which it was originally obtained for use as a therapeutic device.
2. A device according to item 1, wherein the stabilised fluidised bed is an expanded bed.
3. A device according to item 1 or 2, wherein the adsorption particles are contained in a column designed for expanded bed adsorption chromatography having at least one inlet and at least one outlet.
4. A device according to item 1, wherein the stabilised fluidised bed comprises a magnetically stabilised fluidised bed.
5. A device according to item 1, wherein the adsorption particles are contained in a column designed for magnetically stabilised fluidised bed having at least one inlet and at least one outlet.
6. A device according to item 1, wherein the adsorption particles are specially designed for use in a magnetically stabilised fluidised bed.
7. A device according to any of the preceding items in which the number of theoretical plates per m sedimented bed height is at least 5.
8. A device according to any of the preceding items wherein adsorption particles which are characterised by having a specific affinity for the specific bio-macromolecular entities, and having a density of at least 1.3 g/ml and a mean diameter in the range of 5-1000 µm.
9. A device according to any of the preceding items, wherein the contacting of the extracellular body fluid with the adsorption particles is conducted in a stirred or rotated container.
10. A device according to any of the preceding items, wherein the contacting of the extracellular body fluid with the adsorption particles is conducted in a stabilised fluidised bed.
11. A device according to any of the preceding items, wherein the adsorption particles are conglomerate particles comprised of a polymer matrix into which at least two density controlling particles have been incorporated.
12. A device according to any of the preceding items, wherein the adsorption particles have a structure that is pellicular.
13. A device according to any of the preceding items, wherein the average diameter of the adsorption particles is in the range of 10-60 µm, such as in the range of 12-49 µm, preferably in the range of 20-40 µm.
14. A device according to any of the preceding items, wherein at least 95% of the adsorption particles have a diameter in the range of 5-80 µm, such as 15-45 µm, preferably in the range of 20-40 µm.
15. A device according to any of the preceding items, wherein the density of the adsorption particles is at least 1.3, such as at least 2.0, preferably at least 3.0, more preferably at least 3.5, most preferred at least 4 g/ml.
16. A device according to any of the preceding items, wherein the extracellular body fluid is blood.
17. A device according to any of the preceding items, wherein all of steps i), ii), and iii) are performed in a continuous process which is not part of a centrifugation process.
18. A device according to any of the preceding items, wherein the adsorption particles comprises pendant groups such as chargeable pendant groups or affinity specific molecules for bio-macromolecular entities.
19. A device according to item 18, wherein the chargeable pendant groups are selected from the group consisting of polyethyleneimine, modified polyethyleneimine, poly(ethyleneimine/oxyethylene), quaternary aminoethyl (QAE), diethylaminoethyl (DEAE), sulfonic acid, phosphonic acid, and carboxylic acid.
20. A device according to item 18, wherein the affinity specific molecules for bio-macromolecular entities are selected from the group consisting of interchelating affinity specific molecules, hydrophobic affinity specific molecules, specific binding gelatins, albumins, hemoglobulins, immunoglobulins including poly- and monoclonal antibodies, antigens, synthetic peptides, protein G, protein A, blood group specific oligosaccharides, lectins, glycoproteins, biotin binding proteins, avidin and streptavidin, enzymes, proteases, various receptors and protease inhibitors, sequence specific affinity specific molecules , and other bio-catalysts.
21. A device according to item 20, wherein the affinity specific molecules for bio-macromolecular entities are selected from the group consisting of immunoglobulins including poly- and mono-clonal antibodies, antigens, synthetic peptides, blood group specific oligosaccharides, lectins, receptors, and sequence specific affinity specific molecules.
22. A device according to item 21, wherein the affinity specific molecules for bio-macromolecular entities are selected from the group consisting of immunoglobulins including poly- and mono-clonal antibodies, antigens, and lectins.
23. A device according to item 22, wherein the affinity specific molecules for bio-macromolecular entities are antibodies.
24. A device according to any of the preceding items, wherein the captured specific bio-macromolecular entity has a molecular weight of at least 1,000 D, such as at least 5,000 D, preferably at least 100,000 D and most preferably at least 5,000,000 D.
25. A device according to item 24, wherein the bio-macromolecular entity is a cell selected from the group consisting of cancer cells and precancerous cells.
26. A device according to item 24, wherein the bio-macromolecular entity is a parasite selected from the group consisting of single cellular parasites and multi-cellular parasites.
27. A device according to item 24, wherein the bio-macromolecular entity is a virus selected from the group consisting of HIV, encephalitis virus, hepatitis B virus, and hepatitis C virus.
28. A device according to item 27, wherein the virus is HIV.
29. A device according to item 21, wherein the bio-macromolecular entity is a prion.
30. A device according to item 29, wherein the prion is selected from the group of prions that cause Creutzfeldt-Jakob disease, new variant Creutzfeldt-Jakob disease, Gerstmann-Sträussier-Scheinker disease, fatal familial insomnia, kuru, scrapie, bovine spongiform encephalopathy or chronic wasting disease of mule deer and elk.
31. A device according to item 24, wherein the bio-macromolecular entity is an antibody.
32. A device according to any of items 1-31, wherein the column allows for an expansion ratio of 1.2-3.0.
33. The use of a stabilised fluidised bed for the preparation of a device for the continuous therapeutic treatment of a mammalian extracellular body fluid obtained from the mammal by extracorporeal capturing of specific bio-macromolecular entities from the extracellular body fluid, said device comprises (i) an inlet tube for obtaining the mammalian extracellular body fluid, (ii) the stabilised fluidised bed comprising adsorption particles characterised by having a specific affinity for specific bio-macromolecular entities and (iii) an outlet tube for reinfuseing the treated extracellular body fluid into the same mammal as that from which is was originally obtained.
34. The use according to item 33, wherein the stabilised fluidised bed is an expanded bed.
35. The use according to any one of items 33 or 34, wherein the adsorption particles are contained in a column designed for expanded bed adsorption chromatography having at least one inlet and at least one outlet.
36. The use according to item 33, wherein the stabilised fluidised bed comprises a magnetically stabilised fluidised bed.
37. The use according to item 33, wherein the adsorption particles are contained in a column designed for magnetically stabilised fluidised bed having at least one inlet and at least one outlet.
38. The use according to item 33, wherein the adsorption particles are specially designed for use in a magnetically stabilised fluidised bed.
39. The use according to items 33-38 in which the number of theoretical plates per m sedimented bed height is at least 5.
40. The use according to items 33-39, wherein adsorption particles which are characterised by having a specific affinity for the specific bio-macromolecular entities, and having a density of at least 1.3 g/ml and a mean diameter of in the range of 5-1000 µm.
41. The use according to items33-40, wherein the contacting of the extracellular body fluid with the adsorption particles is conducted in a stirred or rotated container.
42. The use according to items 33-41, wherein the contacting of the extracellular body fluid with the adsorption particles is conducted in a stabilised fluidised bed.
43. The use according to items 33-42, wherein the adsorption particles are conglomerate particles comprised of a polymer matrix into which at least two density controlling particles have been incorporated.
44. The use according to items 33-43, wherein the adsorption particles have a structure that is pellicular.
45. The use according to items 33-44, wherein the average diameter of the adsorption particles is in the range of 10-60 µm, such as in the range of 12-49 µm, preferably in the range of 20-40 µm.
46. The use according to items 33-45, wherein at least 95% of the adsorption particles have a diameter in the range of 5-80 µm, such as 15-45 µm, preferably in the range of 20-40 µm.
47. The use according to items 33-46, wherein the density of the adsorption particles is at least 1.3, such as at least 2.0, preferably at least 3.0, more preferably at least 3.5, most preferred at least 4 g/ml.
48. The use according to items 33-47, wherein the extracellular body fluid is blood.
49. The use according to items 33-48, wherein all of steps i), ii), and iii) are performed in a continuous process which is not part of a centrifugation process.
50. The use according to items 33-49, wherein the adsorption particles comprises pendant groups such as chargeable pendant groups or affinity specific molecules for bio-macromolecular entities.
51. The use according to item 50, wherein the chargeable pendant groups are selected form the group consisting of polyethyleneimine, modified polyethyleneimine, poly(ethyleneimine/oxyethylene), quaternary aminoethyl (QAE), diethylaminoethyl (DEAE), sulfonic acid, phosphonic acid, and carboxylic acid.
52. The use according to item 50, wherein the affinity specific molecules for bio-macromolecular entities are selected from the group consisting of interchelating affinity specific molecules, hydrophobic affinity specific molecules, specific binding gelatins, albumins, hemoglobulins, immunoglobulins including poly- and monoclonal antibodies, antigens, synthetic peptides, protein G, protein A, blood group specific oligosaaccharides, lectins, glycoproteins , biotin binding proteins, avidin and streptavidin, enzymes, proteases, various receptors and protease inhibitors, sequence specific affinity specific molecules , and other bio-catalysts.
53. The use according to item 52, wherein the affinity specific molecules for bio-macromolecular entities are selected from the group consisting of immunoglobulins including poly- and mono-clonal antibodies, antigens, synthetic peptides, blood group specific oligosaccharides, lectins, receptors, and sequence specific affinity specific molecules.
54. The use according to item 53, wherein the affinity specific molecules for bio-macromolecular entities are selected from the group consisting of immunoglobulins including poly- and mono-clonal antibodies, antigens, and lectins.
55. The use according to item 54, wherein the affinity specific molecules for bio-macromolecular entities are antibodies.
56. The use according to items 33-55, wherein the captured specific bio-macromolecular entity have a molecular weight of at least 1,000 D, such as at least 5,000 D, preferably at least 100,000 D and most preferably at least 5,000,000 D.
57. The use according to item 56, wherein the bio-macromolecular entity is a cell selected from the group consisting of cancer cells and precancerous cells.
58. The use according to item 56, wherein the bio-macromolecular entity is a parasite selected from the group consisting of single cellular parasites and multi-cellular parasites.
59. The use according to item 56, wherein the bio-macromolecular entity is a virus selected from the group consisting of HIV, encephalitis virus, hepatitis B virus, and hepatitis C virus.
60. The use according to item 59, wherein the virus is HIV.
61. The use according to item 53, wherein the bio-macromolecular entity is a prion.
62. The use according to item 61, wherein the prion is selected from the group of prions that cause Creutzfeldt-Jakob disease, new variant Creutzfeldt-Jakob disease, Gerstmann-Sträussler-Scheinker disease, fatal familial insomnia, kuru, scrapie, bovine spongiform encephalopathy or chronic wasting disease of mule deer and elk.
63. The use according to any of items 33-62, wherein the column allows for an expansion ratio of 1.2-3.0.

## Claims

1. A method for extracorporeal capturing of specific bio-macromolecular entities from an extracellular body fluid, said method comprising the steps of:
(a) providing the extracellular body fluid which has been obtained from a mammal,
(b) *capturing the specific bio-macromolecular entities by* contacting the provided extracellular body fluid with a stabilised fluidised bed of adsorption particles **characterized by** having a specific affinity for the specific bio-macromolecular entities,
(c) optionally, reducing the effect of an anticoagulant, added to the extracellular body fluid,
wherein the treated extracellular body fluid obtained from step (b) or (c) is in a condition to be reinfused into the mammal.

2. A method according to claim 1, wherein the adsorbent particles has an average particle size in the range of 10-75 µm and/or a density in the range of 1.3-20 g/ml

3. A method according to any one of claims 1 or 2, wherein the method is conducted under aseptic conditions.

4. A method according to any one of claims 1-3, wherein the extracellular body fluid is whole blood.

5. A method according to any one of the preceding claims, wherein the specific bio-macromolecular entity is selected from the group consisting of microorganisms and cells, such as cancer cells, certain blood cells, foetal cells, parasites, bacteria (both eu- and archaeo-bacteria) or virus.

6. A method according to any one of the preceding claims, wherein the adsorbent particles comprise particles having at least 95% of the particles in the range of 5-80 µm.

7. A method according to any one of the preceding claims, wherein the stabilization of the fluidised bed is obtained by use of adsorption particles having a well-defined size distribution as well as a well-defined density distribution together with a column designed to give an even liquid flow distribution.

8. A method according to any one of the preceding claims, wherein the method does not comprise a continuous centrifugation process.
